(19) Europäisches Patentamt
European Patent Office
Office européen des brevets

(11) EP 0 697 403 A1

(12) DEMANDE DE BREVET EUROPEEN

(43) Date de publication: **21.02.1996 Bulletin 1996/08**

(21) Numéro de dépôt: **95401912.1**

(22) Date de dépôt: **18.08.1995**

(51) Int Cl.$^6$: **C07D 209/42**, A61K 31/395, C07D 405/04, C07D 215/54, C07D 215/48, C07D 217/26, C07D 487/06, C07C 237/22

(84) Etats contractants désignés: **AT BE CH DE DK ES FR GB GR IE IT LI LU MC NL PT SE**

(30) Priorité: **19.08.1994 FR 9410165**

(71) Demandeur: **SANOFI**
**F-75008 Paris (FR)**

(72) Inventeurs:
- **Bras, Jean-Pierre**
  **F-31500 Toulouse (FR)**
- **de Cointet, Paul**
  **F-31400 Toulouse (FR)**
- **Despeyroux, Pierre**
  **F-31120 Portet/Garonne (FR)**
- **Frehel, Daniel, Résidence de l'Autan**
  **F-31100 Toulouse (FR)**
- **Gully, Danielle**
  **F-31600 Muret (FR)**
- **Maffrand, Jean-Pierre**
  **F-31120 Portet/Garonne (FR)**
- **Bignon, Eric**
  **F-31120 Pinsaguel (FR)**

(74) Mandataire: **Le Guen, Gérard et al**
**F-75441 Paris Cédex 09 (FR)**

# (54) Dérivés de glycinamide comme agonistes des récepteurs de la cholecystokinine

(57) La présente invention concerne des composés de formule :

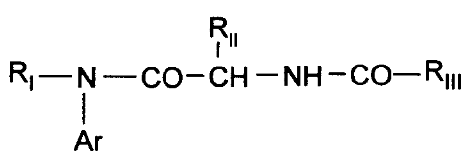

**(I)**

qui sont des agonistes des récepteurs de la cholecystokinine et compositions pharmaceutiques les contenant.

EP 0 697 403 A1

## Description

La présente invention concerne des dérivés du glycinamide, un procédé pour leur préparation et les médicaments les contenant.

Plus particulièrement, la présente invention a pour objet de nouveaux agonistes non peptidiques des récepteurs de la cholécystokinine (CCK).

La CCK est un peptide, qui en réponse à une ingestion d'aliment, est secrétée au niveau périphérique et participe à la régulation de nombreux processus digestifs (CRAWLEY J.N. et al., Peptides, 1994, *15* (4), 731-735).

La CCK a été identifiée par la suite dans le cerveau et pourrait être le neuropeptide le plus abondant agissant comme neuromodulateur des fonctions cérébrales par stimulation des récepteurs de type CCK-B (CRAWLEY J.N. et al., Peptides, 1994, *15* (4), 731-735). Dans le système nerveux central, la CCK interagit avec la transmission neuronale médiée par la dopamine (CRAWLEY J.N. et al., ISIS Atlas of Sci., Pharmac., 1988, 84-90). Elle intervient également dans des mécanismes impliquant l'acétylcholine, le gaba (acide 4-aminobutyrique), la sérotonine, les opioïdes, la somatostatine, la substance P et dans les canaux ioniques.

Son administration provoque des modifications physiologiques : ptose palpébrale, hypothermie, hyperglycémie, catalepsie, et comportementales : hypolocomotricité, diminution de l'exploration, analgésie, modification de la faculté d'apprentissage, modification du comportement sexuel et satiété.

La CCK exerce son activité biologique par l'intermédiaire d'au moins deux types de récepteurs : les récepteurs CCK-A localisés principalement en périphérie, et les récepteurs CCK-B présents essentiellement dans le cortex cérébral. Les récepteurs CCK-A de type périphérique sont aussi présents dans certaines zones du système nerveux central incluant l'area postrema, le noyau du tractus solitaire et le noyau interpédonculaire (MORAN T.H. et al., Bran Research, 1986, *362*, 175-179 ; HILL D.R. et al., J. Neurosci.,1990, *10*, 1070-1081) ; avec cependant des différences d'espèce (HILL D.R. et al., J. Neurosci.,1990, *10*, 1070-1081 ; MAILLEUX P. et al., Neurosci. Lett., 1990, *117*, 243-247 ; BARRETT R.W. et al., Mol. Pharmacol., 1989, *36*, 285-290 ; MERCER J. G. et al., Neurosci. Lett., 1992, *137*, 229-231 ; MORAN T.H. et al., TIPS, 1991, *12*, 232-236).

A la périphérie, par l'intermédiaire des récepteurs CCK-A (MORAN T.H. et al., Brain Research, 1986, *362*, 175-179), la CCK retarde la vidange gastrique, module la motilité intestinale, stimule la contraction vésiculaire, augmente la sécrétion biliaire, contrôle la sécrétion pancréatique (McHUGH P.R. et al., Fed. Proc., 1986, *45*, 1384-1390 ; PENDLETON R.G. et al., J. Pharmacol. Exp. Ther., 1987, *241*, 110-116).

La CCK pourrait agir dans certains cas sur la pression artérielle et influencer les systèmes immunitaires.

Le rôle de la CCK dans le signal de satiété est supporté par le fait que les concentrations plasmatiques de CCK, dépendantes de la composition des repas (fortes concentrations de protéines ou de lipides), sont, après les repas supérieures à celles observées avant les repas (IZZO R.S. et al., Regul. Pept., 1984, *9*, 21-34 ; PFEIFFER A. et al., Eur. J. Clin. Invest., 1993, *23*, 57-62 ; LIEVERSE R.J., Gut 35 501,1994). Des taux plasmatiques de CCK significativement élevés ont été décrits chez des patients anorexiques et/ou boulimiques (PHILIPP E. et al., Life Sci., 1991, *48*, 2442-2450 ; GERACIOTTI T.D. Jr. et al., N. Engl. J. Med., 1988, *319*, 683-688). Chez les boulimiques, il y a une diminution de la sécrétion de la CCK induite par un repas et une baisse des concentrations de CCK dans le liquide céphalo-rachidien... 

Basé sur ces évidences du rôle clé de la CCK dans le signal de satiété périphérique, l'utilité d'agonistes et d'antagonistes de la CCK comme médicament dans le traitement de certains troubles du comportement alimentaire, de l'obésité, et du diabète est indiscutable. Un agoniste des récepteurs de la CCK peut aussi être utilisé en thérapeutique dans le traitement des troubles du comportement émotionnel, sexuel et mnésique (ITOH S. et al., Drug. Develop. Res., 1990, *21*, 257-276), de la schizophrénie, des psychoses (CRAWLEY J.N. et al., ISIS Atlas of Sci., Pharmac., 1988, 84-90 et CRAWLEY J.N., TIPS, 1991, *12*, 232-265), de la maladie de Parkinson, des dyskinésies tardives et de divers troubles de la sphère gastrointestinale (Drugs of the Future, 1992, *17* (3), 197-206).

Des agonistes du récepteur de la CCK sont décrits dans la littérature. Par exemple, certains produits ayant de telles propriétés sont décrits dans EP-A-0383690 et WO 90/06937.

La plupart des agonistes CCK-A décrits à ce jour sont de nature peptidique. Ainsi, le FPL 14294 dérivé de la CCK-7, un puissant agoniste CCK-A non sélectif vis-à-vis des récepteurs CCK-B, possède une puissante activité inhibitrice de la prise de nourriture chez le rat et chez le chien après administration intranasale (SIMMONS R.D. et al., Pharmacol. Biochem. Behav., 1994, *47* (3), 701-708 ; KAISER E.F. et al., FASEB, 1991, *5*, A864). De même, il a été montré que, le A-71623, un tétrapeptide agoniste sélectif des récepteurs CCK-A, est efficace dans des modèles d'anorexie sur une période de 11 jours et entraîne une réduction significative de la prise de poids par rapport au contrôle chez les rongeurs et les singes cynomologues (ASIN K.E. et al., Pharmacol. Biochem. Behav., 1992, *42*, 699-704). De la même façon, des analogues structuraux du A 71623, possédant une bonne efficacité et sélectivité pour les récepteurs CCK-A sont dotés d'une puissante activité anorexigène chez le rat (ELLIOTT R.L. et al., J. Med. Chem., 1994, *37*, 309-313 ; ELLIOTT R.L. et al., J. Med. Chem., 1994, *37*, 1562-1568).

La demande de brevet WO 91/13874 décrit une série de dérivés de la glycinamide possédant une affinité pour les

récepteurs CCK. Plus particulièrement, ces composés sont décrits comme des antagonistes sélectifs du récepteur CCK-B/gastrine, (XIIth Int. Symp. Med. Chem., Bâle, 1992).

On a maintenant trouvé de façon surprenante qu'une série de dérivés de glycinamide possède une puissante activité agoniste des récepteurs CCK-A.

Les composés selon l'invention ont fait l'objet d'études systématiques pour caractériser :

- leur potentialité pour déplacer la [$^{125}$I]-CCK de ses sites de liaison présents sur des membranes pancréatiques de rat (récepteur CCK-A) ou de cellules 3T3 exprimant le récepteur recombinant CCK-A humain,

- leur sélectivité vis-à-vis du récepteur CCK-B présent sur des membranes de cortex de cobaye, les composés étant des ligands sélectifs ou non des récepteurs CCK-A,

- leur propriété agoniste des récepteurs CCK-A à travers leur capacité à induire *in vitro* la sécrétion d'amylase par des cellules pancréatiques chez le rat, ou à provoquer *in vivo* la vidange de la vésicule biliaire chez la souris, ou à bloquer toujours *in vivo* la vidange gastrique chez la souris,

- leur effet sur la consommation alimentaire chez le rat.

Ainsi, la présente invention concerne des composés de formule :

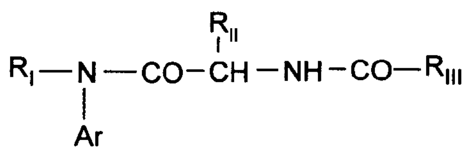

(I)

dans laquelle

- $R_I$ représente un alkyle en $C_3$ à $C_8$ ; un arylalkyle -Alk-$Ar_1$ où Alk représente un alkylène de 1 à 4 atomes de carbone et $Ar_1$ représente un groupe phényle ou un hétérocycle éventuellement substitué par un halogène, un alkyle en $C_1$-$C_3$, un alcoxy en $C_1$-$C_3$, un trifluorométhyle ou un hydroxyle ; un cycloalkylalkyle dans lequel l'alkyle est en $C_1$-$C_4$ et le cycloalkyle en $C_3$-$C_{10}$ ; un cycloalkyle en $C_3$-$C_{10}$ éventuellement substitué par un hydroxyle, un alcoxy en $C_1$-$C_3$ ou un alkyle en $C_1$-$C_3$ ledit alkyle pouvant substituer deux fois le même atome de carbone ; un alcoxyalkyle dans lequel l'alcoxy est en $C_1$-$C_4$ et l'alkyle est en $C_2$-$C_5$ ; ou un groupe (AB)N-CO-$(CH_2)_r$-, où A est un alkyle en $C_1$-$C_3$, B est un alkyle en $C_1$-$C_3$ ou un phényle ou bien A et B forment, avec l'atome d'azote auquel ils sont liés, un hétérocycle choisi parmi pyrrolidine, pipéridine et morpholine, r est 1, 2 ou 3 ;

- $R_{II}$ représente l'hydrogène ; un alkyle en $C_1$-$C_6$ ; un hydroxyalkyle en $C_1$-$C_5$ ; un groupe -$(CH_2)_m$-$COR_2$ dans lequel m est un entier de 1 à 3 et $R_2$ représente un hydroxyle, un groupe alcoxy en $C_1$-$C_4$, un groupe benzyloxy, un groupe -$NR_3R_4$ dans lequel $R_3$ ou $R_4$ représentent indépendamment l'hydrogène, un alkyle en $C_1$-$C_4$ ou constituent avec l'atome d'azote auquel ils sont liés un hétérocycle choisi parmi pyrrolidine, pipéridine et morpholine ; un groupe aralkyle -$(CH_2)_n$-$Ar_2$ dans lequel n est égal à 0 ou représente un entier de 1 à 4 et $Ar_2$ représente un phényle ou un hétérocycle éventuellement substitué par un halogène, un alkyle en $C_1$-$C_3$, un alcoxy en $C_1$-$C_3$, un trifluorométhyle, un hydroxyle ou par un benzyloxy ; un cycloalkylalkyle dans lequel l'alkyle est en $C_1$-$C_4$ et le cycloalkyle en $C_3$-$C_{10}$ ; un aminoalkyle en $C_1$-$C_4$ ; un groupe R-CO-NH-$(CH_2)_x$- dans lequel x représente un nombre entier de 1 à 4 et R représente un alkyle en $C_1$-$C_4$, un phényle, un benzyle, un 2-phényléthényle ou un benzyloxy, les noyaux aromatiques étant éventuellement substitués par un halogène, un alkyle en $C_1$-$C_3$, un alcoxy en $C_1$-$C_3$, un trifluorométhyle, un hydroxyle, un groupe sulfonique ou carboxylique ; un guanidinoalkyle en $C_1$-$C_4$ ; un imidazolylalkyle en $C_1$-$C_3$ ; un alkylthioalkyle dans lequel les alkyles sont en $C_1$-$C_3$ ; un aralkylthioalkyle dans lequel la partie aryle est éventuellement hétérocyclique et les parties alkyle sont en $C_1$-$C_3$, l'aryle étant éventuellement substitué par un halogène, un alkyle en $C_1$-$C_3$, un alcoxy en $C_1$-$C_3$, un trifluorométhyle, un hydroxyle ; un benzyloxyalkyle dans lequel l'alkyle est en $C_1$-$C_3$ et le phényle éventuellement substitué par un halogène, un hydroxyle, un alcoxy en $C_1$-$C_3$, un alkyle en $C_1$-$C_3$, un trifluorométhyle, un nitrile, un nitro ;

- $R_{III}$ représente un groupe naphtyle ; un groupe quinoléinyle ; un groupe isoquinoléinyle ; un groupe indolyle non substitué, substitué sur un carbone, ou substitué sur l'azote par un groupe alkylcarbonyle en $C_1$-$C_4$, par un groupe -$(CH_2)_p$-$COR_5$, p étant un entier de 0 à 4 et $R_5$ représentant $OR'_5$ ou $NR'_5R''_5$ avec $R'_5$ et $R''_5$ identiques ou non représentant l'hydrogène ou un alkyle en $C_1$-$C_4$ ou bien $R'_5$ et $R''_5$ formant ensemble avec l'atome d'azote auquel

ils sont liés une pipéridine, par un hydroxyalkyle en $C_1$-$C_4$, par un alcoxyalkyle en $C_2$-$C_6$, par un cyanoalkyle en $C_2$-$C_4$, par un tétrahydropyranyle, par un adamantylaminocarbonylalkyle en $C_1$-$_4$ ou par une chaîne $-(CH_2)_q-$, q étant un entier de 2 à 4 dont un des carbones substitue le noyau phényle du groupe indolyle pour constituer un cycle ;

- Ar représente un groupe 2-méthoxy-3-pyridinyle, 4-méthoxy-5-pyrimidinyle ou 2-méthoxyphényle contenant au moins deux autres substituants choisis parmi un alkyle en $C_1$-$C_3$, un alcoxy en $C_1$-$C_3$, un atome d'halogène et un trifluorométhyle ; ou Ar représente un groupe naphtyle ;

- ou bien $R_I$ et $R_{II}$ constituent ensemble un groupe

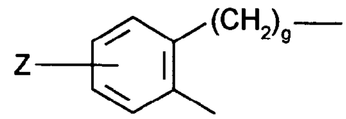

dans lequel g représente 0, 1 ou 2 et Z représente un alkyle en $C_1$-$C_4$, un alcoxy en $C_1$-$C_3$ ou un halogène ; ou éventuellement un de leurs sels.

Une classe de produits avantageux est représentée par la formule suivante:

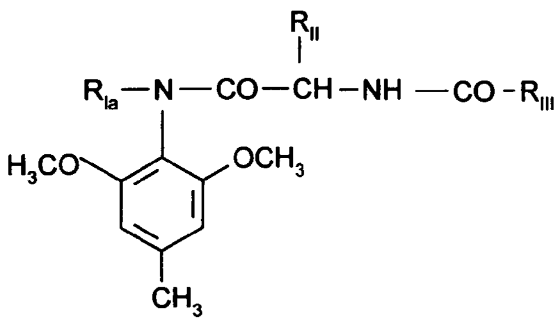

(Ia)

dans laquelle $R_{II}$ et $R_{III}$ sont tels que définis ci-dessus pour (I) et $R_{Ia}$ représente un alkyle en $C_5$-$C_8$ ; un arylalkyle -Alk-$Ar_1$ où Alk représente un alkylène de 1 à 4 atomes de carbone et $Ar_1$ représente un groupe phényle ou un hétérocycle éventuellement substitué par un halogène, un alkyle en $C_1$-$C_3$, un alcoxy en $C_1$- $C_3$, un trifluorométhyle ou un hydroxyle; un cycloalkylalkyle dans lequel l'alkyle est en $C_1$-$C_4$ et le cycloalkyle en $C_3$-$C_{10}$; un cycloalkyle en $C_3$-$C_{10}$ éventuellement substitué par un alcoxy en $C_1$-$C_3$, un hydroxyle ou un alkyle en $C_1$-$C_3$; ledit alkyle pouvant substituer deux fois le même atome de carbone; un alcoxyalkyle dans lequel l'alcoxy est en $C_1$-$C_4$ et l'alkyle est en $C_2$-$C_5$; ou un groupe (AB)N-CO-$(CH_2)_r$-, où A est un alkyle en $C_1$-$C_3$, B est un alkyle en $C_1$-$C_3$ ou un phényle ou bien A et B forment, avec l'atome d'azote auquel ils sont liés, un hétérocycle choisi parmi pyrrolidine, pipéridine et morpholine, et r est 1, 2 ou 3 ou éventuellement un de leur sels.

Les sels d'addition de ces composés sont ceux obtenus le cas échéant, avec des acides et des bases minérales ou organiques : les sels non toxiques pharmaceutiquement acceptables sont préférés mais d'autres sels utilisables pour isoler ou purifier les composés de formule (I) sont aussi un objet de l'invention.

Dans les définitions qui précèdent et celles qui suivent, les radicaux alkyles sont en chaînes droites ou ramifiées.

Lorsque $Ar_1$ ou $Ar_2$ représente un hétérocycle, celui-ci est choisi de préférence parmi pyridine, pyrimidine, pyrazine ou pyridazine.

Lorsque $R_{II}$ représente aralkylthioalkyle, le groupe aryle est choisi de préférence parmi phényle pyridine, pyrimidine, pyrazine ou pyridazine.

Dans la formule (I), les atomes d'halogène sont de préférence des atomes de chlore, de brome ou de fluor.

Les composés de formule (I) comportant un ou plusieurs centres asymétriques présentent des formes isomères. Les racémiques et les énantiomères ou stéréoisomères de ces composés font également partie de l'invention.

Lorsque le substituant $R_{II}$ est autre que l'hydrogène, les énantiomères dans lesquels le carbone portant $R_{II}$ est en configuration R sont préférés.

Lorsque $R_I$ et $R_{II}$ constituent ensemble un groupe

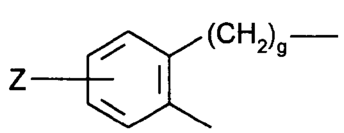

dans lequel Z et g sont tels que définis pour (I), les énantiomères dans lesquels le carbone portant $R_{II}$ est en

configuration S sont préférés.

Un groupe de composés possédant une meilleure activité agoniste des récepteurs CCK-A est celui pour lequel $R_I$ représente un alkyle en $C_3$ à $C_8$, encore meilleur est celui pour lequel $R_I$ représente un alkyle en $C_4$-$C_8$ et préféré est celui pour lequel $R_I$ représente un alkyle en $C_5$-$C_8$, particulièrement préféré est celui pour lequel $R_I$ est un cycloalkyle en $C_5$-$C_7$.

Les composés de formule (I) dans lesquels Ar représente un groupe naphtyle et $R_I$ est $R_{Ia}$ tel que défini ci-dessus forment un autre groupe de composés préférés.

Les composés de formule (I) dans lesquels Ar représente un radical 2-méthoxyphényle substitué par au moins deux substituants, choisis parmi alkyle en $C_1$-$C_4$, alcoxy en $C_1$-$C_3$, un atome d'halogène ou un groupe trifluorométhyle sont des composés avantageux.

Les composés de formule (I) dans lesquels Ar représente un radical 2-méthoxyphényle substitué sur le noyau aromatique par un méthyle et un second méthoxy sont des composés très avantageux.

Les composés de formule (I) dans lesquels $R_{III}$ représente un 2-indolyle N-substitué ou non sont particulièrement avantageux.

Les composés de formule (I) dans lesquels $R_I$ représente un alkyle en $C_5$ ou un cycloalkyle en $C_6$, $R_{II}$ représente un benzyloxyalkyle ou un cycloalkylalkyle, $R_{III}$ représente un 2-indolyle substitué et Ar représente un 2,6-diméthoxy-4-méthylphényle sont plus particulièrement préférés.

Encore plus particulièrement préférés sont les composés suivants :

EXEMPLE 3 : (R)-N-[1-[(2,6-diméthoxy-4-méthylphényl)-pentylcarbamoyl]éthyl]-1*H*-indole-2-carboxamide.

EXEMPLE 6 : Acide 3-[2-[[(cyclohexylméthyl) (2,6-diméthoxy-4-méthylphényl)carbamoyl]méthylcarbamoyl] indol-1-yl]propionique

EXEMPLE 8 : N-{[(2,6-diméthoxy-4-méthylphényl)pentylcarbamoyl]méthylcarbamoyl}-1*H*-indole-2-carboxamide.

EXEMPLE 9 : {2-[[(2,6-diméthoxy-4-méthylphényl)pentylcarbamoyl]méthylcarbamoyl]indol-1-yl}-acétate de méthyle.

EXEMPLE 10 : Acide {2-[[(2,6-diméthoxy-4-méthylphényl)pentyl carbamoyl]méthylcarbamoyl]indol-1-yl}-acétique.

EXEMPLE 13 : Acide (R) -[2-{1-[(2,6-diméthoxy-4-méthylphényl)pentyl]carbamoyl]éthyl}carbamoyl]indol-1-yl]acétique.

EXEMPLE 16 : Acide (R) -4-{(2,6-diméthoxy-4-méthylphényl) pentylcarbamoyl}-4-[(1*H*-indole-2-carbonyl)amino] butyrique.

EXEMPLE 19 : (R)-N-{1-1(2,6-diméthoxy-4-méthylphényl)pentylcarbamoyl]-2-(4-hydroxyphényl)éthyl} -1*H*-indole-2-carboxamide.

EXEMPLE 28 : Acide (R)-4-[(1-carboxyméthyl-1*H*-indole-2-carbonyl)amino]4-[[(2,6-diméthoxy-4-méthylphényl) pentyl]carbamoyl]butyrique.

EXEMPLE 30 : Acide (R){2-[{1-{(2,6-diméthoxy-4-méthylphényl) pentyl-carbamoyl}-2-phényléthyl}carbamoyl] indol-1-yl}acétique.

EXEMPLE 31 : Acide (R)[2-{[1-{(2,6-diméthoxy-4-méthylphényl) pentyl-carbamoyl}-2-(4-hydroxyphényl))éthyl]carbamoyl}indol-1-yl]acétique.

EXEMPLE 32 : Acide (R)[2-{[2-(carbamoyl)-1-{(2,6-diméthoxy-4-méthylphényl)pentylcarbamoyl}éthyl]-carbamoyl} indol-1-yl]acétique.

EXEMPLE 33 : Acide (R)[2-{[3-(carbamoyl)-1-{(2,6-diméthoxy-4-méthylphényl)pentylcarbamoyl}propyl]carbamoyl} indol-1-yl]-acétique.

EXEMPLE 44 : (R)[2-{[1-{(2,6-diméthoxy-4-méthylphényl) pentylcarbamoyl}-2-(benzyloxy)}éthyl]carbamoyl} indol-1-yl]acétate de sodium.

EXEMPLE 51 : (R)-{2-[{1-[[N-(2,6-diméthoxy-4-méthylphényl)pentyl]-carbamoyl]-2-(cyclohexyl)éthyl}-carbamoyl]-indol-1-yl}acétate de sodium.

EXEMPLE 81 : Acide (R)-{2-[{1-[N-(2,6-diméthoxy-4-méthylphényl) pentylcarbamoyl]-2-(benzylthio)éthyl}-carbamoyl]-indol-1-yl}acétique.

EXEMPLE 82 : Acide (R)-{2-[{1-[N-(2,6-diméthoxy-4-méthylphényl) pentylcarbamoyl]-3-(phényl)propyl}carbamoyl] indol-1-yl}acétique.

EXEMPLE 85 : Acide [2-{[(6-chloro-2,4-diméthoxy-5-méthylphényl)pentylcarbamoyl]méthylcarbamoyl}indol-1-yl] acétique.

EXEMPLE 94 : [2-{[(5-chloro-2-méthoxy-4-méthylphényl)pentyl-carbamoyl]méthylcarbamoyl}indol-1-yl]acétate de sodium.

EXEMPLE 103 : Acide [2-{[(2,5-diméthoxy-4-méthylphényl)pentyl carbamoyl]méthylcarbamoyl}indol-1-yl]acétique.

EXEMPLE 109 : N-{[(isopentyl)(2,4,6-triméthoxyphényl)carbamoyl] méthyl}-1*H*-indole-2-carboxamide.

EXEMPLE 112 : [2-{[(benzyl) (2,6-diméthoxy-4-méthylphényl) carbamoyl]méthylcarbamoyl}indol-1-yl]acétate de sodium.

EXEMPLE 118 : Acide [2-{[(cyclohexylméthyl) (2,6-diméthoxy-4-méthylphényl)carbamoyl]méthylcarbamoyl}-indol-1-yl]acétique.

EXEMPLE 126 : N-{[(2,6-diméthoxy-4-méthylphényl)pentyl-carbamoyl]méthyl}-2-naphtalènecarboxamide.

EXEMPLE 127 : N-{[(2,6-diméthoxy-4-méthylphényl)pentyl-carbamoyl]méthyl}-3-quinoléinecarboxamide.

EXEMPLE 137 : 8-{[(2,6-diméthoxy-4-méthylphényl) (3méthoxypropyl)]-carbamoylméthylcarbamoyl}-5,6-dihydro-4*H* -pyrrolo[3,2,1-ij]-2-quinoléinecarboxamide.

EXEMPLE 138 : {2-[[(1-naphtyl)pentylcarbamoyl]méthylcarbamoyl]-indol-1-yl}-acétate de méthyle.

EXEMPLE 139 : Acide {2-[[(1-naphtyl)pentylcarbamoyl]méthylcarbamoyl]-indol-1-yl}-acétique.

EXEMPLE 141 : Acide (R)-[2{1-[N-(2,6-diméthoxy-4-méthylphényl)N-cyclohexylcarbamoyl] éthylcarbamoyl} indol-1-yl]acétique.

EXEMPLE 155 : Acide (R)-[2{[1-{N-(2,6-diméthoxy-4-méthylphényl)N-cyclohexyl-carbamoyl}-2-(benzyloxy)éthyl]-carbamoyl}indol-1-yl]acétique.

EXEMPLE 168 : Acide (R)-[2{[1-{N-(2,6-diméthoxy-4-méthylphényl)N-cyclohexylcarbamoyl}-2-(cyclohexyl)éthyl]-carbamoyl}indol-1-yl]acétique.

EXEMPLE 171 : Acide (R)-[2{[1-{N-(2,6-diméthoxy-4-méthylphényl)N-cyclohexylméthylcarbamoyl}-2-(cyclohexyl) éthyl]-carbamoyl}indol-1-yl]acétique.

EXEMPLE 172 : Acide (R)-{2-[{1-[N-(2,6-diméthoxy-4-méthylphényl) butylcarbamoyl]-2-(benzyloxy)éthyl}-carbamoyl]-indol-1-yl}acétique.

EXEMPLE 192 : Acide (R)-{2-[N-{1-[(2,6-diméthoxy-4-méthylphényl) pentyl-carbamoyl]-5-(cinnamoylamino)pentyl}-carbamoyl]-indole-1-yl}acétique.

Les composés selon l'invention sont préparés selon le SCHEMA réactionnel suivant :

## SCHEMA 1

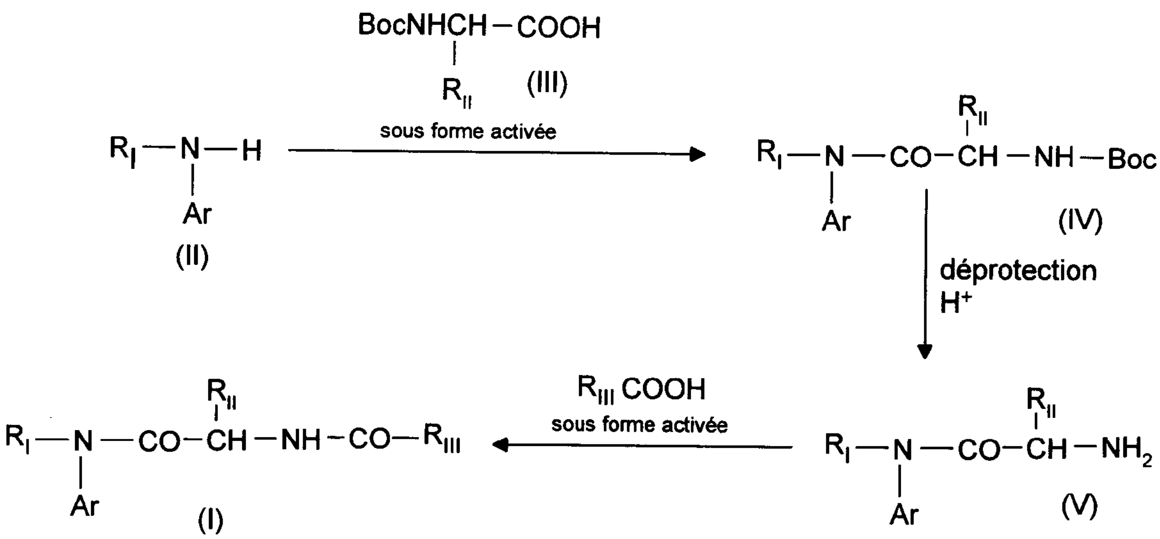


(III) (II) (IV) (V) (I)

Selon un autre de ses aspects, la présente invention concerne un procédé pour la préparation des composés de formule (I) caractérisé en ce que l'on traite une amine de formule :

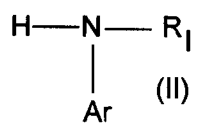

(II)

dans laquelle Ar et $R_I$ sont tels que définis ci-dessus, avec un aminoacide N-protégé de formule :

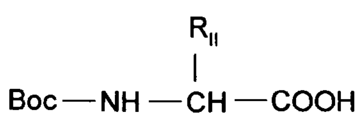

(III)

dans laquelle $R_{II}$ est tel que défini pour (I) et dans laquelle, le cas échéant, les fonctions réactives de $R_{II}$ ont été protégées pour conduire à un composé de formule :

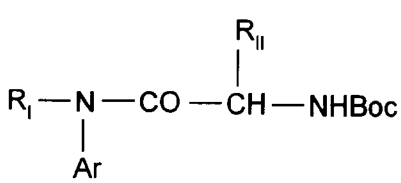

(IV)

dans laquelle $R_I$, Ar et $R_{II}$ sont tels que définis ci-dessus, pour conduire à un composé (I) selon l'invention ou un de ses sels.

Les composés de départ de formules (II) sont soit disponibles commercialement soit préparés selon des méthodes connues, par exemple:

- pour la 2,6-diméthoxy-4-méthylaniline selon une adaptation du procédé décrit par Mori S. et al., Tet. Lett., 1984, *25*, 429 ;
- pour la 2,4-diméthoxy-5-méthylaniline selon Sargent M.V., J. Chem. Soc. Perkin Trans I, 1982, 1095 ;

- pour la 2,4,6-triméthoxyaniline selon EP-A-088849 ;
- pour la 2,5-diméthoxy-4-méthylaniline selon Shaikh Y.A., J. Heterocyclic Chem., 1977, *14*, 1049 ;
- pour la 2,6-diméthoxy-4-trifluorométhylaniline selon une adaptation de procédé décrit par Mori S. et al., Tet. Lett., 1984, *25*, 429 à partir du 1,5-diméthoxy-3-trifluorométhylbenzène préparé selon Robertson A. et al., J. Chem. Soc., 1951,2013 ;
- pour la 4-chloro-2,6-diméthoxyaniline selon Hodgson H. et al., J. Chem. Soc., 1934, 1433 ;
- pour la 1-amino-2,6-diméthoxy-4-méthylpyrimidine selon Urban R. et al., Helv. Chim. Acta., 1958, *41*, 1806 ;
- pour le composé de formule :

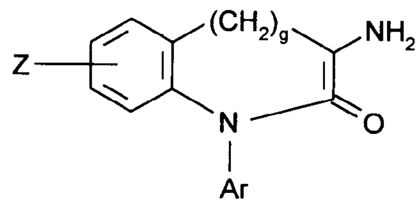

selon EP-0572235.

Lorsque $R_I$ est autre qu'un cycloalkyle, les anilines de formule (II) ou les composés de formule (IV) dans lesquelles Ar est tel que défini pour (I) peuvent être préparées selon des méthodes connues selon le SCHEMA 2 suivant :

## SCHEMA 2

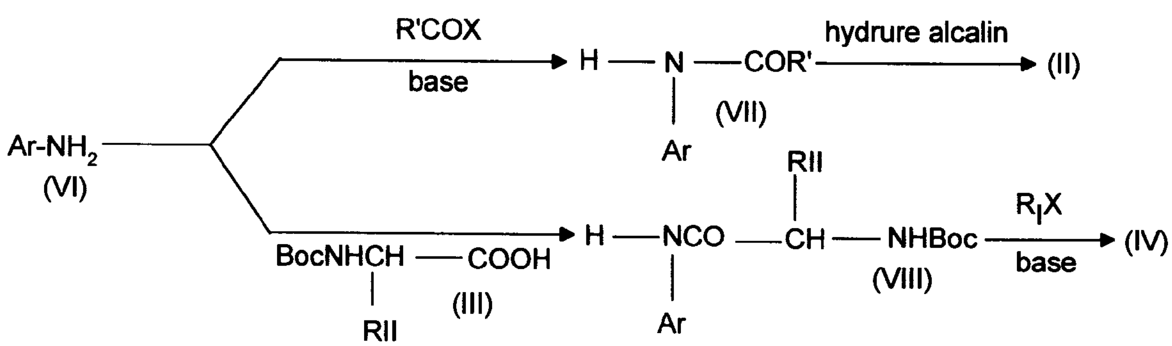


- soit par acylation d'un composé de formule :

$$Ar\text{-}NH_2 \qquad (VI)$$

dans laquelle Ar est tel que défini pour (I) avec un halogénure d'acide de formule R'-CO-X dans lequel X représente un atome de chlore ou de brome et R' est tel que R'-$CH_2$- représente $R_I$ tel que défini pour (I), ou avec l'un de ses esters activés en présence d'une base organique telle que la triéthylamine, la N-éthylmorpholine, dans des solvants organiques tels que l'éther diéthylique, le dichlorométhane, le chloroforme, le diméthylformamide ou le tétrahydrofurane pour conduire à un composé de formule :

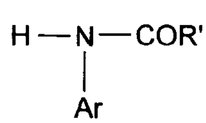

(VII)

dans laquelle R' et Ar sont tels que définis ci-dessus pour le réduire ensuite par un hydrure alcalin tel que l'hydrure de lithium et d'aluminium, dans un solvant organique inerte tel que l'éther diéthylique ou diisopropylique ou le tétrahydrofurane, pour conduire à un composé de formule (II),

- soit par couplage du composé (VI) avec un amino-acide N - protégé préalablement activé de formule (III) pour conduire à un composé de formule :

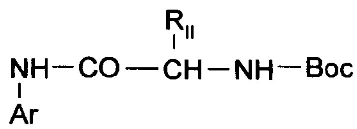

(VIII)

dans laquelle $R_{II}$ et Ar sont tels que définis pour (I) lequel est ensuite N-alkylé après génération de l'anion par une base forte pour obtenir un composé de formule :

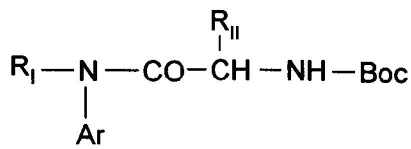

(IV)

dans laquelle $R_I$, $R_{II}$ et Ar sont tels que définis pour (I), ce composé étant alors traité en milieu acide anhydre pour fournir un composé (V) sous forme de sel de formule :

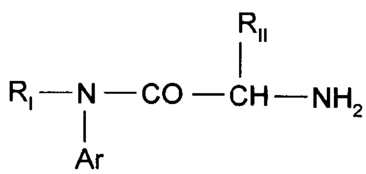

(V)

lequel est alors acylé avec un acide de formule $R_{III}COOH$ préalablement activé pour conduire à un composé (I) selon l'invention ou l'un de ses sels éventuels.

Lorsque $R_I$ représente un cycloalkyle, les anilines de formule (II) sont originales et peuvent être préparées indépendamment selon l'un des SCHEMAS 3, 4 ou 5 ci-après.

## SCHEMA 3

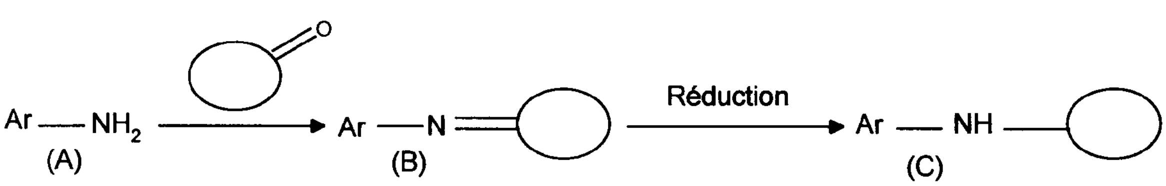


(A) (B) (C)

La condensation de l'aniline (A) dans laquelle Ar est tel que défini pour (I) avec la cycloalcanone conduit à la base de Schiff (B) qui est ensuite réduite selon les conditions habituelles, par exemple par action d'un borohydrure de sodium dans l'éthanol ou par action de l'acide formique à reflux.

## SCHEMA 4

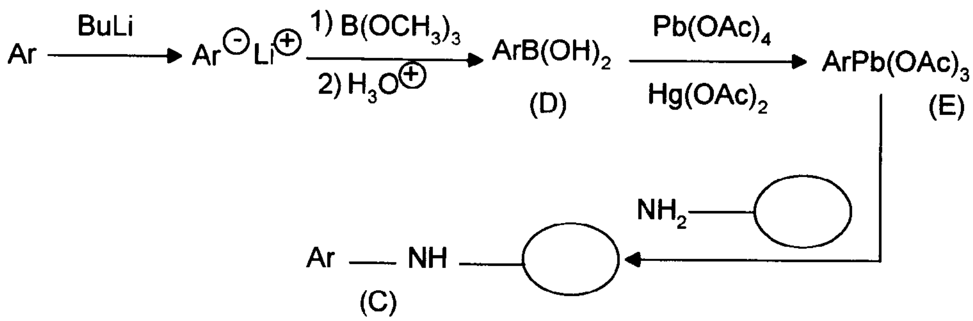


Cette synthèse est une adaptation du procédé décrit par J.T. Pinkey et al., J. Chem. Soc. Perkin Trans I, 1990, 715 pour la préparation des composés (D) et (E) et du procédé décrit par D.H.R. Barton et al., Tet. Lett., 1987, *28* (27), 3111.

## SCHEMA 5

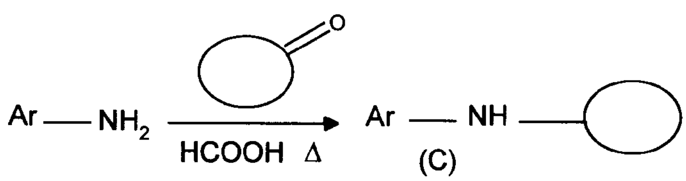


Les acides aminés utilisés sont activés par les réactifs de couplage utilisés couramment en chimie peptidique par exemple, dans le cas d'aminoacides racémiques ou dépourvus de centre d'asymétrie : BOP/$NEt_3$, BOP-Cl/$NEt_3$, DCC/HOBT/$NEt_3$, anhydride mixte avec ClCOOiBu en présence de triéthylamine et dans le cas d'énantiomères R ou S d'aminoacides en présence de BOP/N-éthylmorpholine, ou $Boc_2O$/pyridine.

Les anions des composés (VIII) sont générés par des bases fortes telles que par exemple l'hydrure de sodium ou le tertiobutylate de potassium dans un solvant anhydre aprotique tel que le tétrahydrofurane.

Les composés (V) sont obtenus à partir des acétanilides (IV) en milieu acide anhydre tel que l'acide trifluoroacétique dans le dichlorométhane ou l'acide chlorhydrique gazeux en solution dans l'acétate d'éthyle par exemple.

Les composés (V) sont alors isolés sous forme de chlorhydrate ou de trifluoroacétate, par exemple.

Les composés (I) sont obtenus par les méthodes de couplage peptidiques classiques entre les composés (V) et les acides $R_{III}COOH$ préalablement activés sous forme d'halogénure d'acide, sous forme d'anhydride mixte avec par exemple, ClCOOiBu, sous forme d'ester activé avec BOP/$NEt_3$, BOP/N-éthylmorpholine, BOP-Cl/$NEt_3$, DCC/HOBT/$NEt_3$, $Boc_2O$/pyridine, selon les méthodes bien connues de l'homme de l'art.

Les sels éventuels des composés de formule (I) avec des acides ou des bases organiques ou minérales sont préparés de la façon habituelle par introduction de l'acide, ou de la base dans une solution du composé de formule (I).

Le sel est isolé, selon ses caractéristiques de solubilité, après évaporation du solvant ou addition d'un non-solvant.

Les composés de formule (V) dans laquelle $R_I$, Ar et $R_{II}$ sont tels que définis ci-dessus pour (I) sont nouveaux et constituent un des objets de l'invention.

L'invention a également pour objet selon un autre de ses aspects des compositions pharmaceutiques comprenant les composés (I) ci-dessus.

Plus généralement, les composés de formule (I) ont fait l'objet d'études de liaison *in vitro* concernant les récepteurs CCK.

Une étude de l'effet agoniste des composés sur la sécrétion d'amylase a été réalisée comme suit. Les acini pancréatiques sont obtenus par digestion enzymatique (collagénase) de pancréas de rat soumis à un jeûne de 18 heures. Des aliquotes (485 µl) sont incubées à 37°C pendant 30 minutes en présence de concentrations croissantes d'agoniste selon Jensen et al., J. Biol. Chem., 1982, 257 (10), 5554. L'incubation est arrêtée par une centrifugation de 15 secondes. Le surnageant est conservé dans un bain de glace pour mesurer, le taux d'amylase selon la technique de Ceska et al., Clin. Chim. Acta., 1969, *26*, 437 (réactif phadebas® : test amylase commercialisé par Pharmacia Diagnostic). Les composés à tester sont dissous dans du diméthylsulfoxyde puis dans un tampon d'incubation.

Les composés de formule (I) se comportent comme des agonistes des récepteurs CCK-A avec des $CE_{50}$ (concentration efficace induisant 50 % de la sécrétion d'amylase comparé à l'effet maximum produit en présence de CCK) de l'ordre de $10^{-7}$ à $10^{-9}$ M.

Une étude de l'effet agoniste des composés sur la contraction de la vésicule biliaire a été réalisée comme suit. Les

souris femelles Swiss albinos CD1 (20-25 g) sont mises à jeûn solide pendant 24 heures. Le jour de l'expérience, les produits (en suspension dans une solution de carboxyméthyl cellulose à 1 % ou de méthyl cellulose à 0,6 %) ou le véhicule correspondant sont administrés par voie orale. Les souris sont sacrifiées par dislocation cervicale une heure après l'administration des produits et les vésicules biliaires sont prélevées et pesées. Les résultats sont exprimés en mg/kg de poids corporel (Europ. J. Pharmacol., 1993, *232*, 13-19).

Les composés de formule (I) contractent totalement la vésicule biliaire, comme la CCK elle-même, et se comportent donc comme des agonistes des récepteurs CCK-A. Certains d'entre eux ont des $DE_{50}$ (dose efficace induisant 50 % de la diminution du poids des vésicules observée avec la CCK) inférieure à 3 mg/kg par la voie orale.

Une étude de l'effet agoniste des composés sur la vidange gastrique a été réalisée comme suit. Les souris femelles Swiss albino CD1 (20-25 g) sont mises à jeun solide pendant 18 heures. Le jour de l'expérience, les produits (en suspension dans une solution de carboxyméthyl cellulose à 1 % ou de méthyl cellulose à 0,6 %) ou le véhicule correspondant sont administrés par voie intrapéritonéale 30 minutes avant l'administration d'un repas de charbon (0,3 ml par souris d'une suspension dans l'eau de 10 % de poudre de charbon, 5 % de gomme arabique et 1 % de carboxylméthyl cellulose). Les souris sont sacrifiées 5 minutes plus tard par dislocation cervicale et la vidange gastrique est définie comme la présence de charbon dans l'intestin au delà du sphincter pylorique (Europ. J. Pharmacol., 1993, *232*, 13-19).

Les composés de formule (I) bloquent complètement la vidange gastrique, comme la CCK elle-même, et se comportent donc comme des agonistes des récepteurs CCK. Certains d'entre eux ont des $DE_{50}$ (dose efficace induisant 50 % de l'effet de la CCK) inférieures à 1 mg/kg par la voie intrapéritonéale.

Une étude de l'effet agoniste CCK des composés sur la consommation alimentaire a été réalisée comme suit. Les rats mâles (200-240 g) Sprague Dawley (Charles River, France), sont isolés 10 jours avant l'expérience, et soumis chaque jour successivement à 18 heures de jeûne et 6 heures d'alimentation : la nourriture est disponible de 10 heures à 16 heures, l'eau est disponible *ad libitum*. Le jour de l'expérience, les produits (en suspension dans une solution de méthylcellulose à 0,6 %) ou le véhicule sont administrés par voie intra-péritonéale.

Trente minutes après le traitement (à 10 heures) une quantité connue de nourriture est introduite dans la cage : on mesure la consommation alimentaire 1 heure et 3 heures après.

Les composés de formule (I) diminuent la prise de nourriture et se comportent donc comme des agonistes des récepteurs CCK-A (Gibbs J. et al., J. Comp. Physiol. Psychol., 1973, *84*, 488-495).

Certains d'entre eux sont actifs à la dose de 3 mg/kg, par voie orale, dose à laquelle ils réduisent la consommation alimentaire de 30 à 40 % par rapport à un animal témoin.

Par conséquent, les composés de formule (I) sont utilisés, en tant qu'agonistes des récepteurs de la CCK-A, pour la préparation de médicaments destinés à combattre les maladies dont le traitement nécessite une stimulation par agonisme total ou partiel des récepteurs de la cholécystokinine, plus particulièrement pour la fabrication de médicaments destinés au traitement de certains troubles du comportement alimentaire, de l'obésité, du diabète, des troubles du comportement émotionnel, sexuel et mnésique, des psychoses et notamment la schizophrénie, de la maladie de Parkinson, de la dyskinésie tardive et de divers troubles de la sphère gastrointestinale.

Les composés de formule (I) sont peu toxiques ; leur toxicité est compatible avec leur utilisation comme médicament pour le traitement des troubles et des maladies ci-dessus.

Les composés de formule (I), peuvent être formulés dans des compositions pharmaceutiques pour l'administration aux mammifères, y compris l'homme, pour le traitement des maladies susdites.

Les composés de formule (I) ci-dessus et leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg.

Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention, le principe actif est généralement formulé en unités de dosage contenant de 0,5 à 1000 mg, avantageusement de 1 à 500 mg, de préférence de 2 à 200 mg dudit principe actif par unité de dosage.

La présente invention a donc également pour objet les compositions pharmaceutiques qui contiennent à titre de principe actif un des composés ci-dessus. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec

un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Le principe actif peut être également présenté sous forme de complexe avec une cyclodextrine, par exemple α, β ou γ cyclodextrine, 2-hydroxypropyl-β-cyclodextrine ou méthyl-β-cyclodextrine.

Dans ce qui suit, on décrit des EXEMPLES de mise en oeuvre de l'invention, ainsi que les préparations de certains intermédiaires de synthèse de formule (II), (IV), (V), (VII) et (VIII). Les points de fusion, indiqués ont été déterminés en capillaire.

**PREPARATION I :** ***Composé 1 - Intermédiaire de formule (II)***

(II) : Ar = 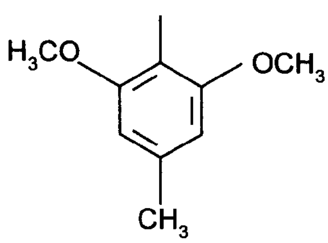 ; $R_I$ = 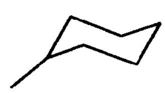

**Etape 1**

On dissout 4,2 g de (2,6-diméthoxy-4-méthyl)aniline dans 50 ml de toluène puis on ajoute 2,45 g de cyclohexanone et chauffe le mélange réactionnel à reflux pendant 18 heures. L'eau qui se forme est éliminée à mesure de sa formation dans le mélange réactionnel au moyen d'un appareillage de Dean-Stark. On évapore le toluène et le résidu huileux obtenu de N-cyclohexylidène (2,6-diméthoxy-4-méthyl)aniline est utilisé sans autre purification dans l'étape suivante.

**Etape 2**

a) Réduction de l'alkylidène par l'acide formique :

On dissout l'alkylidène obtenu précédemment dans 50 ml de toluène et y ajoute 1,15 g d'acide formique goutte à goutte, sous atmosphère inerte. On chauffe le mélange réactionnel à reflux sous atmosphère inerte pendant 4 heures. Après refroidissement on verse le mélange réactionnel dans 100 ml d'une solution aqueuse 2N d'hydroxyde de sodium puis on extrait par de l'acétate d'éthyle. Les extraits organiques sont séchés sur sulfate de sodium anhydre et évaporés à sec. L'huile résiduelle est purifiée par chromatographie-flash sur colonne de gel de silice, éluant : dichlorométhane/méthanol 98/2 (v/v) pour fournir une huile, Rendement : 80 %. L'huile est transformée en chlorhydrate par addition d'une solution de gaz chlorhydrique 5N dans l'éther diéthylique, cristaux blancs, F = 199°C (chlorhydrate).

b) Réduction de l'alkylidène par $NaBH_4$

On dissout l'alkylidène obtenu dans l'étape 1 dans 50 ml d'éthanol et y ajoute par petites portions, sous atmosphère inerte, 0,95 g de borohydrure de sodium, et abandonne le mélange réactionnel à température ambiante pendant 2 heures. On ajoute 20 ml d'acétone au mélange réactionnel et évapore à sec. On reprend le résidu par de l'eau et extrait la phase aqueuse par le dichlorométhane. Les extraits organiques sont séchés sur du sulfate de sodium anhydre et évaporés à sec. Le résidu huileux est chromatographié sur colonne de gel de silice, éluant : dichlorométhane / méthanol 98/2 (v/v). On transforme l'huile obtenue en chlorhydrate de N-cyclohexyl (2,6-diméthoxy-4-méthyl)aniline, cristaux blancs, F = 199°C (chlorhydrate), Rendement : 85 %.

***Composé 2 - Intermédiaire de formule (II)***

(II) : Ar = 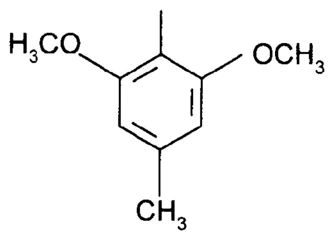 ; $R_I$ = 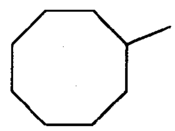

**Etape 1**

A une solution de 73 g de 3,5-diméthoxytoluène dans 450 ml d'éther diéthylique, on ajoute goutte à goutte à température ambiante 300 ml d'une solution (1,6 M) de butyl lithium dans l'hexane. On chauffe le mélange réactionnel au reflux pendant 3 heures sous atmosphère inerte puis on refroidit le mélange réactionnel à -60°C et ajoute goutte à goutte en 60 minutes, 99,7 g de borate de méthyle. On laisse le mélange réactionnel à -60°C pendant 3 heures et laisse remonter à température ambiante. On agite le mélange réactionnel à température ambiante pendant 16 heures puis on ajoute au mélange réactionnel de l'acide chlorhydrique 6N (pH = 1), et laisse décanter. On récupère la phase organique. La phase aqueuse est extraite avec de l'éther diéthylique. Les phases organiques jointes sont séchées sur sulfate de sodium anhydre. L'évaporation du solvant laisse une huile jaune qui cristallise par refroidissement à 0°C. Après séchage on récupère des cristaux blancs de l'acide (2,6-diméthoxy-4-méthylphényl)boronique, F = 108°C, Rendement 80 %.

On met en suspension dans 150 ml de chloroforme anhydre 45,4 g de tétraacétate de plomb et 3,2 g d'acétate mercurique et chauffe le mélange à 40°C sous atmosphère d'argon. On ajoute goutte à goutte, au mélange réactionnel, à 40°C, une solution de 20 g de l'acide (2,6-diméthoxy-4-méthyl)phénylboronique (préparé ci-dessus) dans 100 ml de chloroforme. On laisse 75 minutes à 40°C puis laisse revenir à température ambiante sous bonne agitation. On agite à température ambiante pendant 18 heures puis on dilue le mélange réactionnel avec 800 ml de dichlorométhane. On filtre la solution sur un lit de célite puis on évapore à sec le solvant pour obtenir des cristaux jaunes de (2,6-diméthoxy-4-méthyl)phényl plomb triacétate, F = 172°C, Rendement : 90%.

**Etape 2**

On dissout 10,56 g de cyclooctylamine dans 500 ml de dichlorométhane anhydre et y ajoute 1,5 g d'acétate cuivrique. Au mélange réactionnel, on ajoute, sous atmosphère inerte, goutte à goutte une solution de 44,2 g de (2,6-diméthoxy-4-méthyl)phényl plomb triacétate, préparé ci-dessus, dans 250 ml de dichlorométhane anhydre. On abandonne à température ambiante pendant 18 heures puis on filtre le mélange hétérogène sur un lit de célite et concentre le filtrat à 450 ml. On lave la phase organique avec de l'eau (3 fois). On extrait la phase organique par 3 x 300 ml d'acide chlorhydrique 1N. La phase aqueuse acide est alcalinisée par une solution aqueuse 2N d'hydroxyde de sodium (pH = 11). On extrait la phase aqueuse alcaline par le dichlorométhane et les extraits organiques sont séchés sur sulfate de sodium anhydre. L'évaporation laisse une huile que l'on purifie par chromatographie-flash sur colonne de gel de silice, éluant : dichlorométhane / méthanol : 99/1 (v/v) pour obtenir la N-cyclooctyl(2,6-diméthoxy-4-méthyl)aniline sous forme d'une huile. Rendement 52%.

***Composé 3 - Intermédiaire de formule (II)***

(II) : Ar = 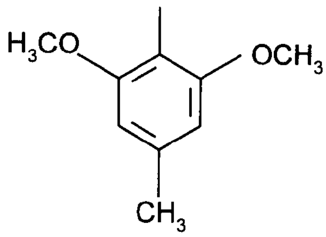 ; $R_I$ = 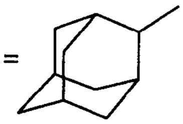

On dissout 2,1 g de (2,6-diméthoxy-4-méthyl)aniline dans 10 ml de toluène puis à cette solution on ajoute 0,5 ml d'acide formique et on chauffe le mélange à léger reflux, sous atmosphère inerte et ajoute à cette température 0,93 g de 2-adamantanone dissout dans 10 ml de toluène, goutte à goutte. On chauffe le mélange réactionnel à reflux pendant 48 heures puis on évapore à sec et reprend le résidu par 30 ml d'acide chlorhydrique 2N. Les cristaux blancs insolubles sont filtrés et écartés. On extrait le filtrat acide avec du dichlorométhane. On lave les extraits organiques avec une solution aqueuse à 5 % de bicarbonate de sodium puis à l'eau, puis on sèche les phases organiques sur sulfate de sodium anhydre. L'évaporation laisse un résidu incolore que l'on purifie par chromatographie-flash sur colonne de gel de silice, éluant : toluène / acétate d'éthyle 7/3 (v/v) pour obtenir la N-(2-adamantyl) (2,6-diméthoxy-4-méthyl)aniline ; F = 100°C, Rendement 52 %.

***Composé 4 - Intermédiaire de formule (II)***

(II) : Ar = 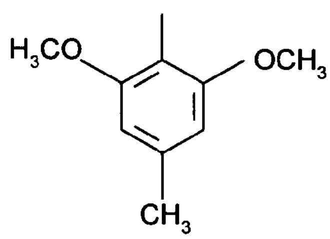 ; $R_I = CH_3$

**Etape 1**

On dissout 3,4 g de (2,6-diméthoxy-4-méthyl)aniline dans 40 ml de toluène. A la solution on ajoute 1,03 g d'acide formique et chauffe le mélange réactionnel à reflux, sous atmosphère inerte, pendant 24 heures. On évapore à sec et reprend le résidu par 40 ml d'acide chlorhydrique 2N puis on agite 30 minutes à température ambiante et filtre le précipité que l'on sèche. On obtient 3,6 g de cristaux blancs de N-formyl(2,6-diméthoxy-4-méthyl)aniline, F = 132°C, Rendement : 90 %.

**Etape 2**

On met en suspension 6 g de N-formyl(2,6-diméthoxy-4-méthyl)aniline dans 100 ml de tétrahydrofurane puis on ajoute goutte à goutte, sous atmosphère inerte, 33 ml d'une solution 1M d'hydrure de lithium et d'aluminium dans le tétrahydrofurane. Le mélange réactionnel devient homogène. On abandonne à température ambiante le mélange réactionnel pendant 2 heures puis après refroidissement à 0°C, on ajoute au mélange réactionnel, goutte à goutte successivement 1 ml d'eau, puis 1 ml d'une solution aqueuse d'hydroxyde de sodium à 15 %, puis 3 ml d'eau. On dilue l'ensemble avec 100 ml d'acétate d'éthyle et filtre le précipité. Le filtrat évaporé à sec laisse un résidu huileux que l'on purifie par chromatographie-flash sur colonne de gel de silice, éluant : toluène/acétate d'éthyle 8/2 (v/v) pour obtenir la N-méthyl (2,6-diméthoxy-4-méthyl)aniline sous forme d'huile, Rendement :85 %.

**PREPARATION II** ***Composé 5 - Intermédiaire de formule (VII)***

(VII) : Ar = 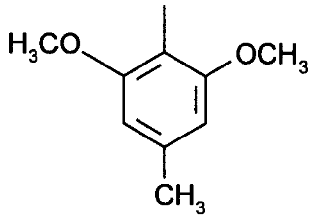 ; $-COR' = -COCH_2CH(CH_3)_2$

On dissout 8,2 g de (2,6-diméthoxy-4-méthyl)aniline dans 100 ml d'éther diéthylique à 0°C sous atmosphère inerte, on ajoute 5,96 g de triéthylamine au mélange réactionnel puis, goutte à goutte, 6,51 g de chlorure d'isovaléryle, en maintenant la température à 0°C. On abandonne le mélange à température ambiante pendant 30 minutes puis le verse dans 250 ml d'eau. On extrait la phase aqueuse avec de l'acétate d'éthyle après décantation de la phase éthérée. Les phases organiques sont jointes et lavées à l'eau puis séchées sur du sulfate de sodium anhydre et évaporées à sec. Les cristaux blancs obtenus de N-(2,6-diméthoxy-4-méthyl)phénylisovaleramide sont lavés avec de l'éther diisopropylique ; F = 139°C, Rendement : 92 %.

En procédant selon la PREPARATION II on prépare les *composés intermédiaires* 6 à 25 décrits ci-après dans le TABLEAU A.

**TABLEAU A** : *Intermédiaires de formule (VII)*

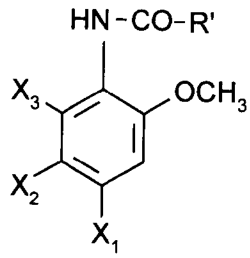

| *Composé* | R' | $X_1$ | $X_2$ | $X_3$ | F ; °C |
|---|---|---|---|---|---|
| *6* | $-(CH_2)_3CH_3$ | $OCH_3$ | $OCH_3$ | H | 72 |
| *7* | $-(CH_2)_3CH_3$ | $CH_3$ | $OCH_3$ | H | 101 |
| *8* | $-(CH_2)_3CH_3$ | $OCH_3$ | $CH_3$ | Cl | 138 |
| *9* | $-(CH_2)_3CH_3$ | Cl | $CH_3$ | H | 90 |
| *10* | $-(CH_2)_3CH_3$ | Cl | $OCH_3$ | H | 113 |
| *11* | $-(CH_2)_3CH_3$ | $CH_3$ | Cl | H | 80 |
| *12* | $-(CH_2)_5CH_3$ | $CH_3$ | H | $OCH_3$ | 94 |
| *13* | $-CH_2-CH(CH_3)_2$ | $OCH_3$ | H | $OCH_3$ | 142 |
| *14* | 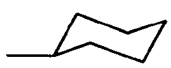 | $CH_3$ | H | $OCH_3$ | 210 |
| *15* | $-(CH_2)_2OCH_3$ | $CH_3$ | H | $OCH_3$ | 116 |
| *16* | 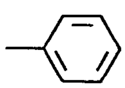 | $CH_3$ | H | $OCH_3$ | 110 |
| *17* | 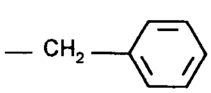 | $CH_3$ | H | $OCH_3$ | 126 |
| *18* | $-(CH_2)_3CH_3$ | (VII) : Ar = 1-naphtyle | | | 113 |

**TABLEAU A** : Suite 1

| *Composé* | R' | $X_1$ | $X_2$ | $X_3$ | F ; °C |
|---|---|---|---|---|---|
| *19* | $-(CH_2)_3CH_3$ | $OCH_3$ | $CH_3$ | H | 91 |
| *20* | $-(CH_2)_3CH_3$ | $OCH_3$ | H | $OCH_3$ | 125 |
| *21* | $-(CH_2)_2CH_3$ | $CH_3$ | H | $OCH_3$ | 123 |
| *22* | 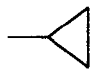 | $CH_3$ | H | $OCH_3$ | 180 |
| *23* | $CH_3$ | $CH_3$ | H | $OCH_3$ | 156 |
| *24* | 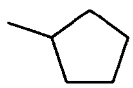 | $CH_3$ | H | $OCH_3$ | 178 |
| *25* | H | $CH_3$ | H | $OCH_3$ | 132 |

**PREPARATION III** ***Composé 26 - Intermédiaire de formule (II)***

(II) : Ar = 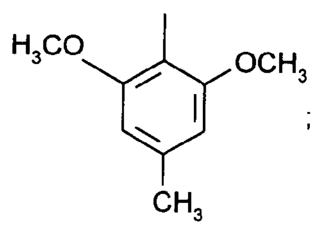 ; $R_I$ = $—(CH_2)_2CH(CH_3)_2$

On dissout 8,5 g de l'anilide (*composé 5*) préparée précédemment dans 160 ml de tétrahydrofurane sec que l'on refroidit à 0°C. On ajoute, goutte à goutte, 34 ml d'une solution 1M d'hydrure de lithium et d'aluminium dans le tétrahydrofurane puis on laisse revenir le mélange réactionnel à température ambiante et le chauffe à reflux pendant 2h 30. On le laisse revenir à température ambiante puis refroidit à 0°C.

A 0°C on ajoute au mélange réactionnel, successivement, 2,5 ml d'eau glacée, 7 ml d'une solution aqueuse d'hydroxyde de sodium 6N puis 2,5 ml d'eau; on sépare l'insoluble par filtration et le lave sur le filtre bûchner avec de l'acétate d'éthyle. Le filtrat est lavé à l'eau salée, séché sur sulfate de sodium anhydre et évaporé sous vide. On récupère une huile jaune qui est purifiée par chromatographie-flash sur colonne de gel de silice, éluant : dichlorométhane/éther diéthylique 98/2 (v/v) pour fournir la N-isopentyl(2,6-diméthoxy-4-méthyl)aniline, sous forme d'huile. Rendement : 89 %.

En procédant selon les PREPARATIONS I et III on prépare *les intermédiaires 27 à 45* décrits ci-après dans le TABLEAU B

**TABLEAU B** : *Intermédiaires de formule (II)*

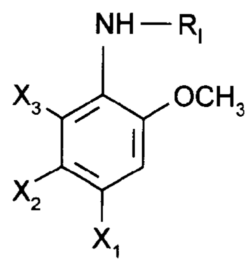

| *Composés* | $R_I$ | $X_1$ | $X_2$ | $X_3$ | F ; °C sel |
|---|---|---|---|---|---|
| *27* | $-(CH_2)_4CH_3$ | $CH_3$ | H | $OCH_3$ | 217, HCl |
| *28* | $-(CH_2)_4CH_3$ | $OCH_3$ | $OCH_3$ | H | huile |
| *29* | $-(CH_2)_4CH_3$ | $CH_3$ | $OCH_3$ | H | 38 |
| *30* | $-(CH_2)_4CH_3$ | $OCH_3$ | $CH_3$ | Cl | huile |
| *31* | $-(CH_2)_4CH_3$ | Cl | $CH_3$ | H | huile |
| *32* | $-(CH_2)_4CH_3$ | Cl | $OCH_3$ | H | 40 |
| *33* | $-(CH_2)_4CH_3$ | $CH_3$ | Cl | H | 64 |
| *34* | $-(CH_2)_6-CH_3$ | $CH_3$ | H | $OCH_3$ | huile |
| *35* | $-(CH_2)_2CH(CH_3)_2$ | $OCH_3$ | H | $OCH_3$ | 180, HCl |
| *36* | 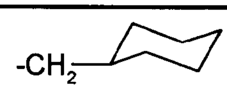 | $CH_3$ | H | $OCH_3$ | huile |
| *37* | $-(CH_2)_3OCH_3$ | $CH_3$ | H | $OCH_3$ | huile |
| *38* | 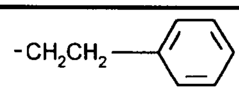 | $CH_3$ | H | $OCH_3$ | huile |
| *39* | $-(CH_2)_4CH_3$ | (II) :Ar = 1-naphtyle | | | huile |

**TABLEAU B** : Suite 1

| ***Composés*** | $R_I$ | $X_1$ | $X_2$ | $X_3$ | F ; °C sel |
|---|---|---|---|---|---|
| *40* | $-(CH_2)_4CH_3$ | $OCH_3$ | $CH_3$ | H | huile |
| *41* | $-(CH_2)_4CH_3$ | $OCH_3$ | H | $OCH_3$ | huile |
| *42* | $-(CH_2)_3CH_3$ | $CH_3$ | H | $OCH_3$ | huile |
| *43* | 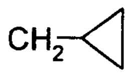 | $CH_3$ | H | $OCH_3$ | huile |
| *44* | 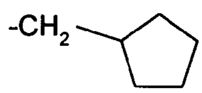 | $CH_3$ | H | $OCH_3$ | huile |
| *45* | $CH_3$ | $CH_3$ | H | $OCH_3$ | huile |

**PREPARATION IV** ***Composé 46 -Intermédiaire de formule (IV)***

(IV) : Ar = 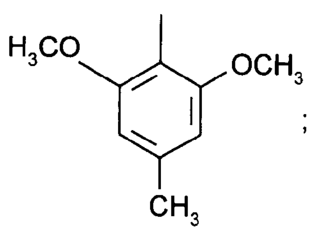 ; $R_I = —(CH_2)_2CH(CH_3)_2$ ;

$R_{II} = H$

Synthèse avec un acide aminé non chiral.

On dissout 5,5 g de l'aniline (*composé 26*) préparée précédemment dans 60 ml de diméthylformamide. On ajoute successivement au mélange réactionnel 10,8 g de BOP, 4,26 g de N-Boc-glycine, puis goutte à goutte 4,69 g de triéthylamine et on abandonne le mélange réactionnel, sous atmosphère inerte, à température ambiante, pendant 20 heures. On verse le mélange réactionnel dans 200 ml d'eau froide et extrait la phase aqueuse avec de l'acétate d'éthyle. La phase organique est lavée à l'eau et séchée sur sulfate de sodium anhydre. L'évaporation du solvant laisse des cristaux blancs qui sont purifiés par chromatographie-flash sur une colonne de gel de silice, éluant : dichlorométhane/éther diéthylique 95/5 (v/v) pour obtenir des cristaux blancs de N-terbutyloxycarbonyl[(2,6-diméthoxy-4-méthylphényl)isopentyl carbamoyl]méthylamine ; F = 132°C ; Rendement : 91 %.

**PREPARATION V** ***Composé 47 - Intermédiaire de formule (VIII)***

(VIII) : Ar = 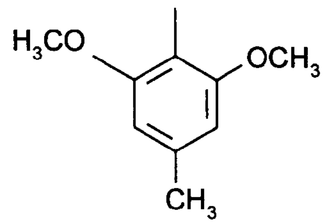 ; $R_{II}$ = H .

Synthèse avec un acide aminé non chiral.

A 100 ml de diméthylformamide, on ajoute successivement 6,6 g de N-Boc-glycine, 15,6 g de BOP, puis à 0°C on ajoute, goutte à goutte, 14 ml de triéthylamine. On abandonne le mélange pendant 20 minutes à 0°C et on additionne ensuite par portions, 6,7 g de chlorhydrate de (2,6-diméthoxy-4-méthyl)aniline. On abandonne le mélange réactionnel à température ambiante pendant 15 heures. On additionne 400 ml d'acétate d'éthyle au mélange réactionnel et on lave successivement la phase organique par 3 x 200 ml d'eau, par une solution d'hydroxyde de sodium aqueuse 1N, puis par de l'eau. On séche la phase organique sur sulfate de sodium anhydre. L'évaporation du solvant laisse un résidu semi-cristallin que l'on concrète dans l'éther diisopropylique pour fournir le N-terbutyloxycarbonyl [(2,6-diméthoxy-4-méthylphényl)carbamoylméthylamine], sous forme de cristaux blancs ; F = 146°C ; Rendement : 96%.

**PREPARATION VI** ***Composé 48 - Intermédiaire de formule (IV)***

(IV) : Ar = 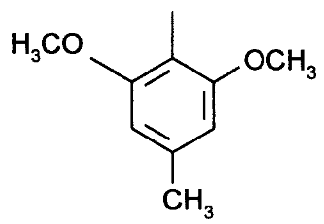 ; $R_{II}$ = $—CH_2CO_2CH_2C_6H_5$ ;

$R_I$ = $—(CH_2)_4—CH_3$ , **énantiomère R**

Synthèse avec un acide aminé chiral.

On dissout 3,2 g de N-pentyl(2,6-diméthoxy-4-méthyl)aniline (*composé 27*) (II) dans 50 ml de diméthylformamide et ajoute successivement à 0°C, 5 g de l'acide N-Boc-aspartiqueβ-O-benzylester, 7,2 g de BOP puis goutte à goutte 1,6 g de N-éthylmorpholine et on abandonne le mélange réactionnel à température ambiante pendant 19 heures. On ajoute ensuite 200 ml d'acétate d'éthyle et lave la phase organique successivement avec 2 x 200 ml d'eau, 100 ml d'une solution d'hydroxyde de sodium 0,1N aqueuse, 100 ml d'une solution d'acide chlorhydrique 0,1N aqueuse et deux fois 200 ml d'eau.

La phase organique est séchée sur sulfate de sodium anhydre et évaporée à sec. L'huile orangée obtenue est purifiée par filtration sur un lit de silice, éluant : dichlorométhane, pour obtenir le 3-(N-terbutyloxycarbonyl)amino-3-([(2,6-diméthoxy-4-méthylphényl)pentyl]carbamoyl}propionate de benzyle sous forme d'huile ; Rendement : 98%.

**PREPARATION VII** ***Composé 49 - Intermédiaire de formule (IV)***

(IV) : Ar = 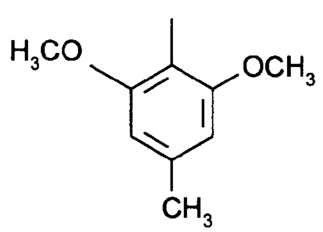 ; $R_I$ = $—CH_2C_6H_5$ ;

$R_{II}$ = H.

On dissout 3,99 g de N-terbutyloxycarbonyl[(2,6-diméthoxy-4-méthylphényl)carbamoylméthylamine] *(composé 47)* dans 50 ml de diméthylformamide et ajoute par portions 0,5 g d'hydrure de sodium en suspension à 60 % dans l'huile,

à 0°C. On agite le mélange réactionnel à température ambiante pendant 30 minutes puis on ajoute, goutte à goutte, au mélange réactionnel 2,16 g de bromure de benzyle en solution dans 30 ml de diméthylformamide. On abandonne le mélange réactionnel à température ambiante pendant 2 heures puis on ajoute 300 ml d'acétate d'éthyle. La phase organique est lavée à l'eau et séchée sur sulfate de sodium anhydre. L'évaporation du solvant laisse un résidu semi-cristallin que l'on concrète dans de l'éther diisopropylique pour obtenir : la N-terbutyloxycarbonyl [benzyl(2,6-diméthoxy-4-méthylphényl)carbamoylméthylamine] sous forme de cristaux blancs, F = 163°C ; Rendement : 85%.

En procédant selon les PREPARATIONS (IV), (VI) et (VII) on prépare les *composés intermédiaires 50 à 99* décrits dans le TABLEAU C ci-après.

**TABLEAU C** : *Intermédiaires de formule (IV)*

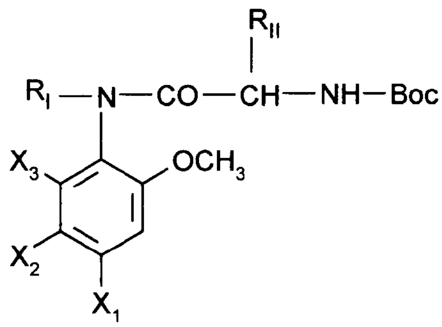

| *Composé N°* | $R_I$ | $R_{II}$ et configuration | $X_1$ | $X_2$ | $X_3$ | F ; °C |
|---|---|---|---|---|---|---|
| *50* | $-(CH_2)_4CH_3$ | H | $CH_3$ | H | $OCH_3$ | 124 |
| *51* | $-(CH_2)_4CH_3$ | H | $OCH_3$ | $CH_3$ | Cl | huile |
| *52* | $-(CH_2)_4CH_3$ | H | $OCH_3$ | $OCH_3$ | H | 174 |
| *53* | $-(CH_2)_4CH_3$ | H | Cl | $CH_3$ | H | huile |
| *54* | $-(CH_2)_4CH_3$ | H | Cl | $OCH_3$ | H | 84 |
| *55* | $-(CH_2)_4CH_3$ | H | $CH_3$ | $OCH_3$ | H | 122 |
| *56* | $-(CH_2)_4CH_3$ | $CH_2CH_2CONH_2$ (R) | $CH_3$ | H | $OCH_3$ | huile |
| *57* | $-(CH_2)_4CH_3$ | $CH_2CONH_2$ (R) | $CH_3$ | H | $OCH_3$ | huile |
| *58* | $-(CH_2)_4CH_3$ | $CH_3$ (R) | $CH_3$ | H | $OCH_3$ | huile |
| *59* | $-(CH_2)_6CH_3$ | H | $CH_3$ | H | $OCH_3$ | 108 |
| *60* | $-(CH_2)_3OCH_3$ | H | $CH_3$ | H | $OCH_3$ | 135 |
| *61* | $-(CH_2)_2CH(CH_3)_2$ | H | $OCH_3$ | H | $OCH_3$ | huile |

**TABLEAU C** : Suite1

| *Composé N°* | $R_I$ | $R_{II}$ et configuration | $X_1$ | $X_2$ | $X_3$ | F ; °C |
|---|---|---|---|---|---|---|
| *62* | 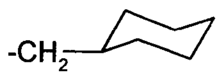 | H | $CH_3$ | H | $OCH_3$ | 191 |
| *63* | 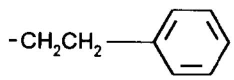 | H | $CH_3$ | H | $OCH_3$ | 170 |
| *64* | $-(CH_2)_4CH_3$ | 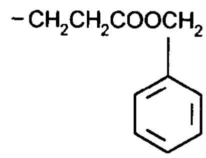 (S) | $CH_3$ | H | $OCH_3$ | huile |
| *65* | $-(CH_2)_4CH_3$ | 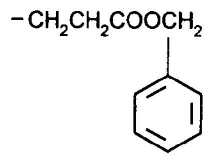 (R) | $CH_3$ | H | $OCH_3$ | huile |
| *66* | $-(CH_2)_4CH_3$ | 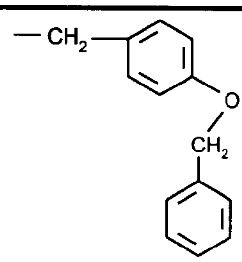 (R) | $CH_3$ | H | $OCH_3$ | huile |
| *67* | $-(CH_2)_4CH_3$ | 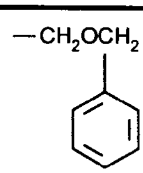 (R) | $CH_3$ | H | $OCH_3$ | 159 |

**TABLEAU C** : Suite 2

| *Composé N°* | $R_I$ | $R_{II}$ et configuration | $X_1$ | $X_2$ | $X_3$ | F ; °C |
|---|---|---|---|---|---|---|
| *68* | $-(CH_2)_4CH_3$ | 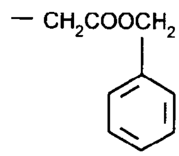 (R) | $CH_3$ | H | $OCH_3$ | huile |
| *69* | $-(CH_2)_4CH_3$ | 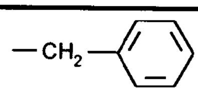 (R) | $CH_3$ | H | $OCH_3$ | huile |
| *70* | $-(CH_2)_4CH_3$ | H | Ar = 1-naphtyle | | | huile |
| *71* | 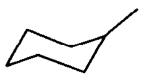 | $-CH_3$(R) | $CH_3$ | H | $OCH_3$ | huile |
| *72* | $-(CH_2)_4CH_3$ | 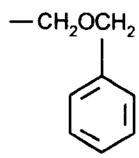 (R) | $OCH_3$ | $CH_3$ | Cl | huile |
| *73* | $-(CH_2)_4CH_3$ | 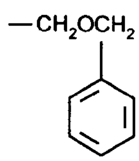 (R) | $OCH_3$ | $CH_3$ | H | huile |
| *74* | 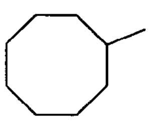 | 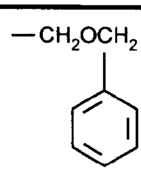 (R) | $CH_3$ | H | $OCH_3$ | huile |
| *75* | $-(CH_2)_4CH_3$ | 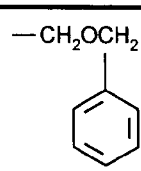 (R) | $CH_3$ | Cl | H | huile |

**TABLEAU C** : Suite 3

| *Composé N°* | $R_I$ | $R_{II}$ et configuration | $X_1$ | $X_2$ | $X_3$ | F ; °C |
|---|---|---|---|---|---|---|
| *76* | 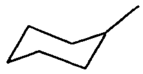 | 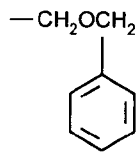 (R) | $CH_3$ | H | $OCH_3$ | huile |
| *77* | 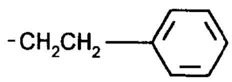 | 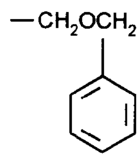 (R) | $CH_3$ | H | $OCH_3$ | huile |
| *78* | $-(CH_2)_4CH_3$ | 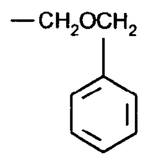 (R) | Cl | $CH_3$ | H | huile |
| *79* | 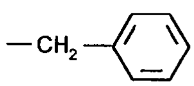 | 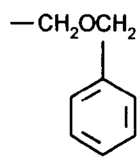 (R) | $CH_3$ | H | $OCH_3$ | huile |
| *80* | $-(CH_2)_3CH_3$ | 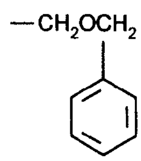 (R) | $CH_3$ | H | $OCH_3$ | huile |
| *81* | $-(CH_2)_4CH_3$ | 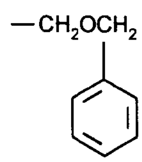 (R) | $OCH_3$ | H | $OCH_3$ | huile |

**TABLEAU C** : Suite 4

| *Composé N°* | $R_I$ | $R_{II}$ et configuration | $X_1$ | $X_2$ | $X_3$ | F ; °C |
|---|---|---|---|---|---|---|
| *82* | 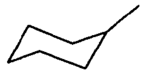 | -$CH_2$ 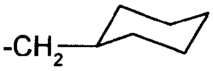 (R) | $CH_3$ | H | $OCH_3$ | huile |
| *83* | -$CH_2$ 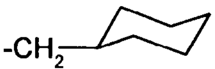 | —$CH_2OCH_2$ 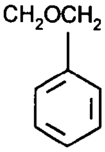 (R) | $CH_3$ | H | $OCH_3$ | huile |
| *84* | -$(CH_2)_4CH_3$ | $CH_3$ (S) | $CH_3$ | H | $OCH_3$ | huile |
| *85* | -$(CH_2)_4CH_3$ | -$CH_2$ 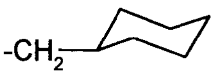 (R) | $CH_3$ | H | $OCH_3$ | huile |
| *86* | -$(CH_2)_4CH_3$ | —$CH_2OCH_2$ 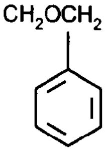 (S) | $CH_3$ | H | $OCH_3$ | huile |
| *87* | -$(CH_2)_4CH_3$ | 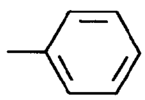 (R, S) | $CH_3$ | H | $OCH_3$ | huile |
| *88* | -$(CH_2)_4CH_3$ | -$CH(CH_3)_2$ (R) | $CH_3$ | H | $OCH_3$ | huile |
| *89* | -$(CH_2)_4CH_3$ | -$(CH_2)_4NHCOO-CH_2$ (R) 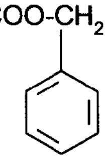 | $CH_3$ | H | $OCH_3$ | huile |

**TABLEAU C** : Suite 5

| *Composé N°* | $R_I$ | $R_{II}$ et configuration | $X_1$ | $X_2$ | $X_3$ | F ; °C |
|---|---|---|---|---|---|---|
| *90* | $-(CH_2)_4CH_3$ | 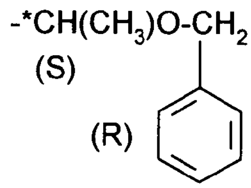 (S) (R) | $CH_3$ | H | $OCH_3$ | huile |
| *91* | $-(CH_2)_4CH_3$ | 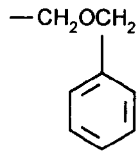 (R) | Cl | $OCH_3$ | H | huile |
| *92* | $-(CH_2)_4CH_3$ | 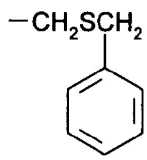 (R) | $CH_3$ | H | $OCH_3$ | huile |
| *93* | $-(CH_2)_4CH_3$ | 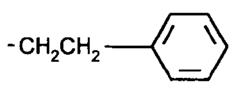 (R) | $CH_3$ | H | $OCH_3$ | huile |
| *94* | 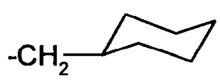 | 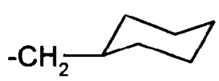 (R) | $CH_3$ | H | $OCH_3$ | huile |
| *95* | $-CH_2CH_3$ | 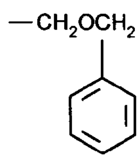 (R) | $CH_3$ | H | $OCH_3$ | huile |

## TABLEAU C : Suite 6

| *Composé N°* | $R_I$ | $R_{II}$ et configuration | $X_1$ | $X_2$ | $X_3$ | F ; °C |
|---|---|---|---|---|---|---|
| *96* | 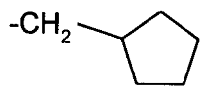 | 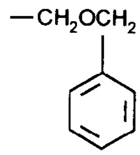 (R) | $CH_3$ | H | $OCH_3$ | huile |
| *97* | $-CH_3$ | 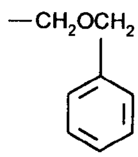 (R) | $CH_3$ | H | $OCH_3$ | huile |
| *98* | 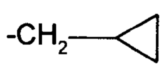 | 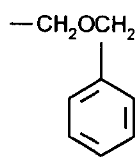 (R) | $CH_3$ | H | $OCH_3$ | huile |
| *99* | 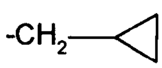 | 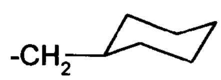 (R) | $CH_3$ | H | $OCH_3$ | huile |

**PREPARATION VIII** ***Composé 100 - Intermédiaire de formule (V)***

(V) : Ar = 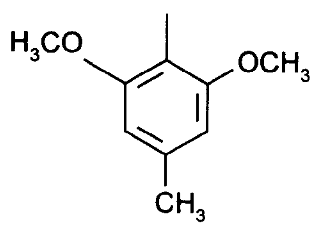 ; $R_I$ = $-(CH_2)_2CH(CH_3)_2$ ;

$R_{II}$ = H.

On dissout 7,8 g d'anilide N-boculé (IV) (*composé 46*) préparé précédemment selon la PREPARATION IV dans 80 ml d'acétate d'éthyle et on refroidit à 0°C. On ajoute au mélange réactionnel 50 ml d'une solution saturée d'acide chlorhydrique gazeux dans l'acétate d'éthyle puis on laisse revenir à température ambiante pendant 2 heures. On évapore à sec l'acétate d'éthyle et reprend le résidu semi-cristallin par de l'éther diéthylique. Les cristaux blancs obtenus sont filtrés et lavés avec de l'éther diéthylique pour fournir des cristaux blancs de chlorhydrate de [(2,6-diméthoxy-4-méthylphényl)isopentyl]carbamoylméthylamine ; F = 214°C ;
Rendement : 96 %.

En procédant selon la préparation VIII, on prepare les *composés intermédiaires 101 à 151* décrits dans le TABLEAU D ci-après.

**TABLEAU D** : *Intermédiaires de formule (V)*

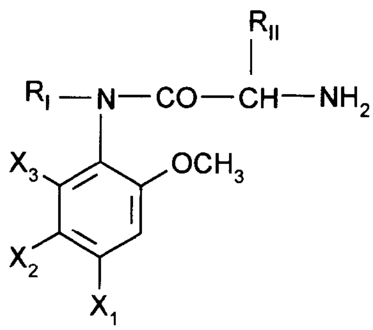

| *Composé N°* | $R_I$ | $R_{II}$ et configuration | $X_1$ | $X_2$ | $X_3$ | F ; °C $[\alpha]_D^{20}$ (c = ; solvant) |
|---|---|---|---|---|---|---|
| *101* | $-(CH_2)_4CH_3$ | H | $CH_3$ | H | $OCH_3$ | 192 |
| *102* | $-(CH_2)_4CH_3$ | H | $OCH_3$ | $CH_3$ | Cl | huile |
| *103* | $-(CH_2)_4CH_3$ | H | $OCH_3$ | $OCH_3$ | H | huile |
| *104* | $-(CH_2)_4CH_3$ | H | Cl | $CH_3$ | H | huile |
| *105* | $-(CH_2)_4CH_3$ | H | Cl | $OCH_3$ | H | huile |
| *106* | $-(CH_2)_4CH_3$ | H | $CH_3$ | $OCH_3$ | H | 161 |
| *107* | $-(CH_2)_4CH_3$ | $-CH_2CH_2CONH_2$ (R) | $CH_3$ | H | $OCH_3$ | 212<br>ND(1) |
| *108* | $-(CH_2)_4CH_3$ | $-CH_2CONH_2$ (R) | $CH_3$ | H | $OCH_3$ | 170<br>ND |
| *109* | $-(CH_2)_4CH_3$ | $CH_3$ (R) | $CH_3$ | H | $OCH_3$ | huile<br>ND |
| *110* | $-(CH_2)_6CH_3$ | H | $CH_3$ | H | $OCH_3$ | 169 |

(1) : ND : $[\alpha]_D$ non déterminé

**TABLEAU D** : Suite 1

| Composé N° | $R_I$ | $R_{II}$ et configuration | $X_1$ | $X_2$ | $X_3$ | F ; °C $[\alpha]_D^{20}$ (c = ; solvant) |
|---|---|---|---|---|---|---|
| 111 | $-(CH_2)_3OCH_3$ | H | $CH_3$ | H | $OCH_3$ | 196 |
| 112 | $-(CH_2)_2CH\ (CH_3)_2$ | H | $OCH_3$ | H | $OCH_3$ | 120 HCl |
| 113 | 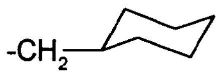 | H | $CH_3$ | H | $OCH_3$ | 207 |
| 114 | 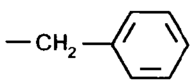 | H | $CH_3$ | H | $OCH_3$ | 190 |
| 115 | 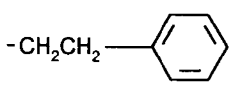 | H | $CH_3$ | H | $OCH_3$ | 215 |
| 116 | $-(CH_2)_4CH_3$ | 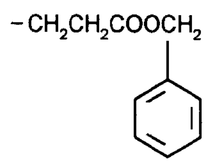 (S) | $CH_3$ | H | $OCH_3$ | huile ND |
| 117 | $-(CH_2)_4CH_3$ | 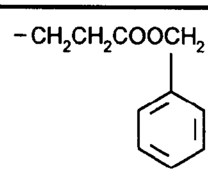 (R) | $CH_3$ | H | $OCH_3$ | huile ND |
| 118 | $-(CH_2)_4CH_3$ | 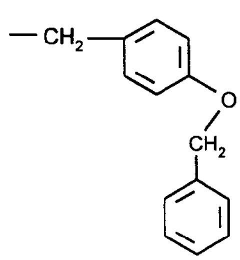 (R) | $CH_3$ | H | $OCH_3$ | 90 ND |

**TABLEAU D** : Suite 2

| Composé N° | $R_I$ | $R_{II}$ et configuration | $X_1$ | $X_2$ | $X_3$ | F ; °C $[\alpha]_D^{20}$ (c = ; solvant) |
|---|---|---|---|---|---|---|
| *119* | $-(CH_2)_4CH_3$ | 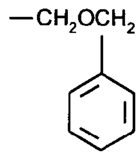 (R) | $CH_3$ | H | $OCH_3$ | huile ND |
| *120* | $-(CH_2)_4CH_3$ | 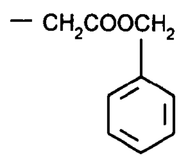 (R) | $CH_3$ | H | $OCH_3$ | huile ND |
| *121* | $-(CH_2)_4CH_3$ | 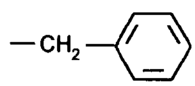 (R) | $CH_3$ | H | $OCH_3$ | huile ND |
| *122* | $-(CH_2)_4CH_3$ | H | Ar = 1-naphtyle | | | 85 |
| *123* | 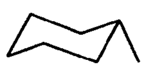 | $-CH_3$ (R) | $CH_3$ | H | $OCH_3$ | huile ND |
| *124* | $-(CH_2)_4CH_3$ | 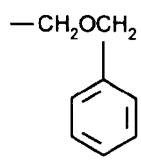 (R) | $OCH_3$ | $CH_3$ | Cl | huile -112,0 (1 ; $CH_3OH$) |
| *125* | $-(CH_2)_4CH_3$ | 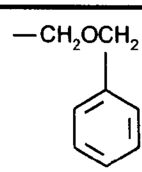 (R) | $OCH_3$ | $CH_3$ | H | huile -3,7 (0,8 ; $CH_3OH$) |

**TABLEAU D** : Suite 3

| Composé N° | $R_I$ | $R_{II}$ et configuration | $X_1$ | $X_2$ | $X_3$ | F ; °C $[\alpha]_D^{20}$ (c = ; solvant) |
|---|---|---|---|---|---|---|
| 126 | 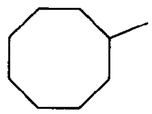 | 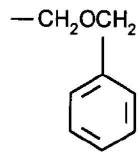 (R) | $CH_3$ | H | $OCH_3$ | huile ND |
| 127 | $-(CH_2)_4CH_3$ | 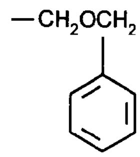 (R) | $CH_3$ | Cl | H | huile ND |
| 128 | 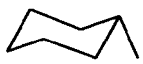 | 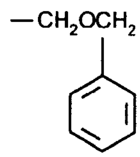 (R) | $CH_3$ | H | $OCH_3$ | huile -3,0 (1 ; $CH_3OH$) |
| 129 | 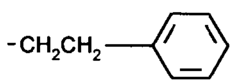 | 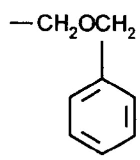 (R) | $CH_3$ | H | $OCH_3$ | huile -25,7 (1,4 ; $CH_3OH$) |
| 130 | $-(CH_2)_4CH_3$ | 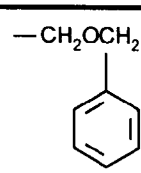 (R) | Cl | $CH_3$ | H | huile +81,0 (1,65 ; $CH_3OH$) |

| | | | | | | |
|---|---|---|---|---|---|---|
| *131* | 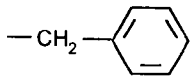 | 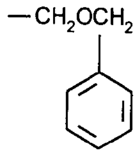 (R) | $CH_3$ | H | $OCH_3$ | huile ND |

**TABLEAU D** : Suite 4

| Composé N° | $R_I$ | $R_{II}$ et configuration | $X_1$ | $X_2$ | $X_3$ | F ; °C $[\alpha]_D^{20}$ (c = ; solvant) |
|---|---|---|---|---|---|---|
| 132 | $-(CH_2)_3CH_3$ | 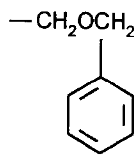 (R) | $CH_3$ | H | $OCH_3$ | huile ND |
| 133 | $-(CH_2)_4CH_3$ | 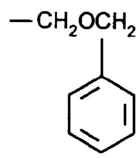 (R) | $OCH_3$ | H | $OCH_3$ | huile -22,1 (1 ; $CH_3OH$) |
| 134 | 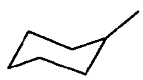 | 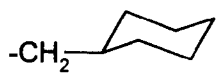 (R) | $CH_3$ | H | $OCH_3$ | huile -10,9 (1,15 ; $CH_3OH$) |
| 135 | 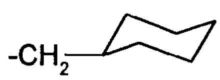 | 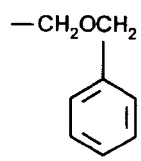 (R) | $CH_3$ | H | $OCH_3$ | huile +16,0 (0,65 ; $CH_3OH$) |
| 136 | $-(CH_2)_4CH_3$ | $-CH_3$ (S) | $CH_3$ | H | $OCH_3$ | huile ND |
| 137 | $-(CH_2)_4CH_3$ | 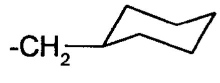 (R) | $CH_3$ | H | $OCH_3$ | huile -33,0 (1,17 ; $CH_3OH$) |

**TABLEAU D** : Suite 5

| Composé N° | $R_I$ | $R_{II}$ et configuration | $X_1$ | $X_2$ | $X_3$ | F ; °C $[\alpha]_D^{20}$ (c = ; solvant) |
|---|---|---|---|---|---|---|
| *138* | $-(CH_2)_4CH_3$ | 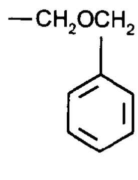 (S) | $CH_3$ | H | $OCH_3$ | huile -174,0 (1,07 ; $CH_3OH$) |
| *139* | $-(CH_2)_4CH_3$ | 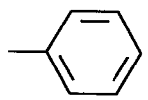 (R, S) | $CH_3$ | H | $OCH_3$ | huile |
| *140* | $-(CH_2)_4CH_3$ | $-CH(CH_3)_2$ (R) | $CH_3$ | H | $OCH_3$ | huile -28,0 (1 ; $CH_3OH$) |
| *141* | $-(CH_2)_4CH_3$ | 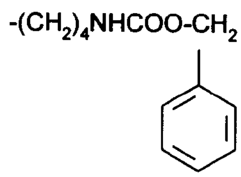 (R) | $CH_3$ | H | $OCH_3$ | huile -32,0 (1 ; $CH_2Cl_2$) |
| *142* | $-(CH_2)_4CH_3$ | 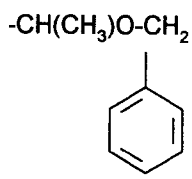 (R) | $CH_3$ | H | $OCH_3$ | huile ND |
| *143* | $-(CH_2)_4CH_3$ | 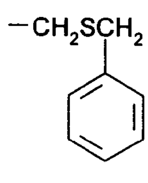 (R) | $CH_3$ | H | $OCH_3$ | huile -21,2 (1 ; $CH_3OH$) |

**TABLEAU D** : Suite 6

| Composé N° | $R_I$ | $R_{II}$ et configuration | $X_1$ | $X_2$ | $X_3$ | F ; °C $[\alpha]_D^{20}$ (c = ; solvant) |
|---|---|---|---|---|---|---|
| 144 | $-(CH_2)_4CH_3$ | $-CH_2CH_2$ 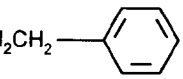 (R) | $CH_3$ | H | $OCH_3$ | huile ND |
| 145 | $-CH_2$ 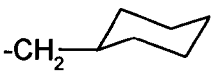 | $-CH_2$ 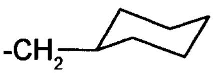 (R) | $CH_3$ | H | $OCH_3$ | 148°C (chlorhydrate) -21,7 (1,05 ; $CH_3OH$) |
| 146 | $-(CH_2)_4CH_3$ | $-CH_2OCH_2$ 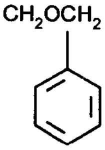 (R) | Cl | $OCH_3$ | H | huile -5,4 (1,45 ; $CH_3OH$) |
| 147 | $-CH_2CH_3$ | $-CH_2OCH_2$ 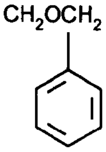 (R) | $CH_3$ | H | $OCH_3$ | huile -25,0 (1,15 ; $CH_3OH$) |
| 148 | $-CH_2$ 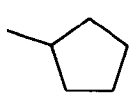 | $-CH_2OCH_2$ 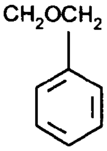 (R) | $CH_3$ | H | $OCH_3$ | huile -47,9 (1 ; $CH_3OH$) |

**TABLEAU D** : Suite 7

| Composé N° | $R_I$ | $R_{II}$ et configuration | $X_1$ | $X_2$ | $X_3$ | F ; °C $[\alpha]_D^{20}$ (c = ; solvant) |
|---|---|---|---|---|---|---|
| *149* | 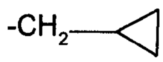 | 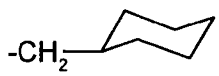 (R) | $CH_3$ | H | $OCH_3$ | huile -17,9 (1 ; $CH_3OH$) |
| *150* | $-CH_3$ | 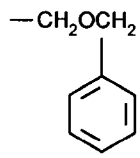 (R) | $CH_3$ | H | $OCH_3$ | huile -13,5 (1 ; $CH_3OH$) |
| *151* | 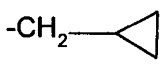 | 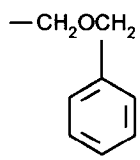 (R) | $CH_3$ | H | $OCH_3$ | huile -30,8 (1 ; $CH_3OH$) |

**EXEMPLE 1**

(I) : Ar = 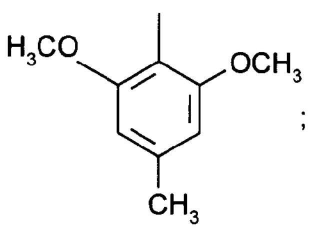 ; $R_I$ = $-(CH_2)_2CH(CH_3)_2$ ; $R_{II}$ = H ;

$R_{III}$ = 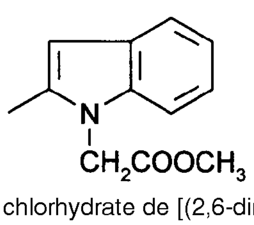

On dissout 0,8 g du chlorhydrate de [(2,6-diméthoxy-4-méthylphényl)isopentyl]carbamoylméthylamine (*composé 100*) dans 10 ml de diméthylformamide et à température ambiante on ajoute successivement au mélange réactionnel 0,58 g de l'acide 1-(méthoxycarbonylméthyl)-2-indolcarboxylique, 1,12 g de BOP puis, goutte à goutte, 0,74 g de triéthylamine. On abandonne le mélange réactionnel à température ambiante pendant 20 heures puis on le verse dans de l'eau froide et extrait la phase aqueuse avec de l'acétate d'éthyle. Les extraits organiques sont lavés à l'eau puis séchés sur sulfate de sodium anhydre. L'évaporation du solvant laisse une huile jaune que l'on purifie par chromatographie-flash sur une colonne de gel de silice, éluant : dichlorométhane/méthanol 98/2 (v/v) pour obtenir des cristaux blancs de {2-[[(2,6-diméthoxy-4-méthylphényl)isopentylcarbamoyl]-méthylcarbamoyl]-1-indolyl}acétate de méthyle ; F = 141°C ; Rendement : 91 %.

**EXEMPLE 2**

(I) : Ar = 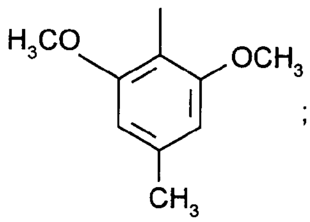 ; $R_I$ = $-(CH_2)_2CH(CH_3)_2$ ; $R_{II}$ = H ;

$R_{III}$ = 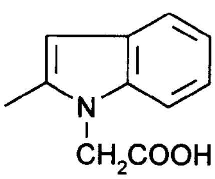

On met en suspension 0,6 g de l'ester préparé selon l'EXEMPLE 1 dans 20 ml de méthanol et additionne au mélange réactionnel 1,8 ml d'une solution d'hydroxyde de sodium aqueuse 1N. On ajoute 6 ml de diméthylformamide pour homogénéiser le mélange réactionnel puis abandonne le mélange réactionnel 2 heures à température ambiante. On évapore le méthanol et verse le résidu dans de l'eau froide. La phase aqueuse est acidifiée avec une solution d'acide chlorhydrique 1N aqueux et extraite par le dichlorométhane. Les extraits organiques sont lavés à l'eau et séchés sur sulfate de sodium anhydre. L'évaporation du solvant laisse des cristaux blancs d'acide {2-[[(2,6-diméthoxy-4-méthylphényl)isopentylcarbamoyl]méthylcarbamoyl]-1-indolyl}acétique qui sont lavés avec de l'éther diisopropylique, F = 208°C ; Rendement : 96 %.

**EXEMPLE 3**

(I) : Ar = 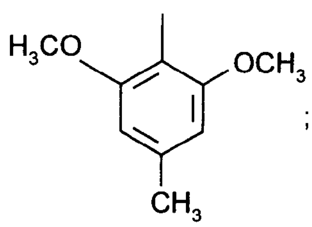 ; $R_I$ = $-(CH_2)_4CH_3$ ; $R_{II}$ = $-CH_3$ ;

$R_{III}$ = 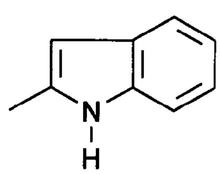 ; **énantiomère** R.

On met en suspension 0,6 g du chlorhydrate de (R)-1-[(2,6-diméthoxy-4-méthylphényl)pentylcarbamoyl]éthylamine (*composé 109*) dans 10 ml de diméthylformamide. A 0°C, on ajoute 0,286 g d'acide 1*H*-indole-2-carboxylique, 0,808 g de BOP et, goutte à goutte, 0,6 g de N-éthylmorpholine et on abandonne le mélange réactionnel à température ambiante pendant 18 heures. On verse le mélange réactionnel dans l'eau froide et extrait la phase aqueuse avec de l'acétate d'éthyle. Les extraits organiques sont lavés à l'eau et séchés sur sulfate de sodium anhydre. L'évaporation du solvant laisse un résidu cristallin brun que l'on purifie par chromatographie sur colonne de gel de silice, éluant : dichlorométhane/éthanol 99/1 (v/v) pour obtenir la (R)-N-{1-[(2,6-diméthoxy-4-méthylphényl)pentylcarbamoyl]éthyl}-1*H*-indole-2-carboxamide, sous forme de cristaux blancs ; F = 193°C ; Rendement : 84 %,

$[\alpha]_D^{20}$ = -78° (c = 1, $CH_2Cl_2$).

**EXEMPLE 4**

(I) : Ar = 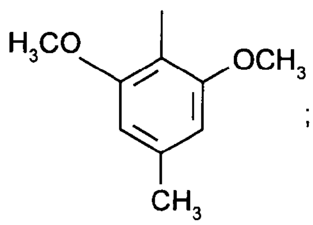 ; $R_I$ = $-CH_2-$ 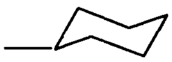 ; $R_{II}$ = H ;

$R_{III}$ = 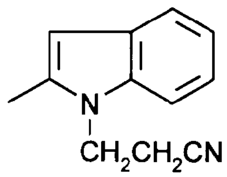

On dissout 1,5 g du chlorhydrate de [(cyclohexylméthyl)(2,6-diméthoxy-4-méthylphényl)]carbamoylméthylamine (*composé 113*) dans 10 ml de diméthylformamide puis on ajoute successivement 0,918 g de l'acide 1 -(2-cyanoéthyl) -2-indolecarboxylique, 1,95 g de BOP, puis goutte à goutte 1,28 g de triéthylamine. On abandonne le mélange réactionnel à température ambiante pendant 3 heures puis on le verse dans l'eau froide et extrait la phase aqueuse avec de l'acétate d'éthyle. Les extraits organiques sont séchés sur sulfate de sodium anhydre et évaporés à sec. Le résidu est purifié par chromatographie-flash sur colonne de gel de silice, éluant : dichlorométhane/méthanol 98/2 (v/v) pour obtenir le 3-{2-[[(cyclohexylméthyl)(2,6-diméthoxy-4-méthylphényl)carbamoyl]méthylcarbamoyl]-1-indolyl}propionitrile sous forme d'une mousse pâteuse ; Rendement : 91 %.

**EXEMPLE 5**

(I) : Ar = 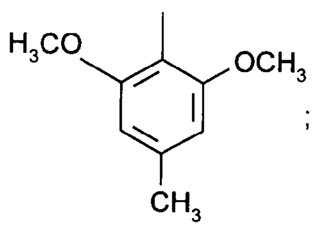 ; $R_I$ = $-CH_2-$ 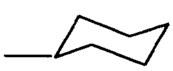 ; $R_{II}$ = H ;

$R_{III}$ = 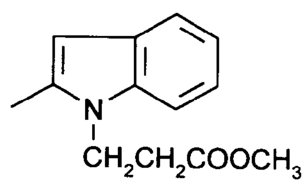

On sature à 0°C (durée 30 minutes) 40 ml de méthanol avec de l'acide chlorhydrique gazeux. On y introduit, goutte à goutte, 1,9 g du nitrile préparé selon l'EXEMPLE 4, préalablement dissous dans 10 ml de méthanol et refroidi à -10°C et on abandonne le mélange réactionnel à -5°C pendant 18 heures. On dégaze puis évapore à sec le méthanol. Le résidu est repris par un mélange d'eau et de méthanol et on abandonne le mélange réactionnel à température ambiante pendant 3 heures. On évapore à sec, reprend le résidu par de l'eau. La phase aqueuse est extraite par de l'acétate d'éthyle. Les extraits organiques sont séchés sur sulfate de sodium anhydre et évaporés sous vide à sec. Le résidu huileux est purifié par chromatographie sur colonne de gel de silice, éluant : dichlorométhane/méthanol 98/2 (v/v) pour obtenir des cristaux blancs, de 3-{2-[[(cyclohexylméthyl)(2,6-diméthoxy-4-méthylphényl) carbamoyl]méthylcarbamoyl] -1-indolyl} propionate de méthyle ; F = 68°C. ; Rendement : 92 %.

**EXEMPLE 6**

(I) : Ar = 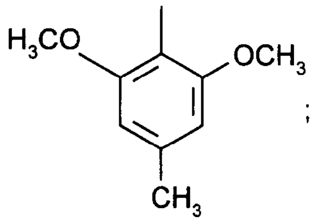 ; $R_I$ = 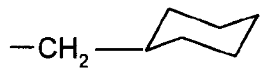 ; $R_{II}$ = H ;

$R_{III}$ = 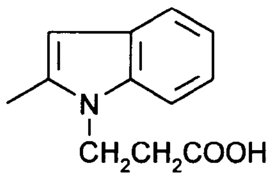

On dissout 1,2 g de l'ester préparé précédemment selon l'EXEMPLE 5 dans 15 ml de méthanol et additionne 3,4 ml d'une solution 1N d'hydroxyde de lithium. On abandonne le mélange réactionnel à température ambiante pendant 18 heures puis on évapore le méthanol et reprend le résidu par de l'eau. La phase aqueuse est extraite par de l'acétate d'éthyle. Les extraits organiques sont séchés sur sulfate de sodium anhydre et évaporés à sec. L'huile incolore est cristallisée dans du pentane pour fournir des cristaux blancs d'acide 3- {2-[[(cyclohexylméthyl)(2,6-diméthoxy-4-méthylphényl) carbamoyl]méthylcarbamoyl]-1-indolyl}propionique ; F = 110°C ;
Rendement : 98 %.

**EXEMPLE 7**

(I) : Ar = 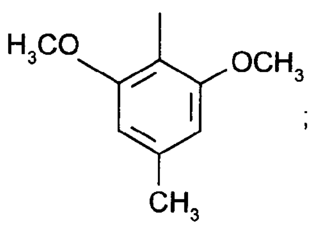 ; $R_I$ = 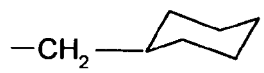 ; $R_{II}$ = H ;

$R_{III}$ = 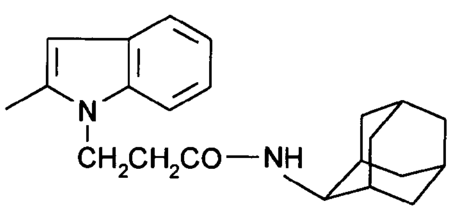

On dissout 0,6 g de l'acide préparé selon l'EXEMPLE 6 dans 10 ml de diméthylformamide puis on ajoute au mélange réactionnel 0,173 g de 2-adamantanamine, 0,505 g de BOP, puis, goutte à goutte, 0,227 g de triéthylamine. On abandonne le mélange réactionnel à température ambiante pendant 18 heures puis on le verse dans l'eau et extrait la phase aqueuse avec de l'acétate d'éthyle. Les extraits organiques sont séchés sur sulfate de sodium anhydre et évaporés à sec. Le résidu obtenu est purifié par chromatographie sur colonne de gel de silice, éluant dichlorométhane/méthanol 99/1 (v/v).

On obtient des cristaux blancs de N-(2-adamantyl)-3[2-{[(cyclohexylméthyl)(2,6-diméthoxy-4-méthylphényl)carbamoyl]méthylcarbamoyl}-1-indolyl] propionamide ; F = 96°C ; Rendement : 88%.

En procédant selon les EXEMPLES 1 à 7 et en utilisant les produits de départ appropriés, on prépare les EXEMPLES 8 à 184 décrits dans les TABLEAUX I à VII ci-après.

## TABLEAU I

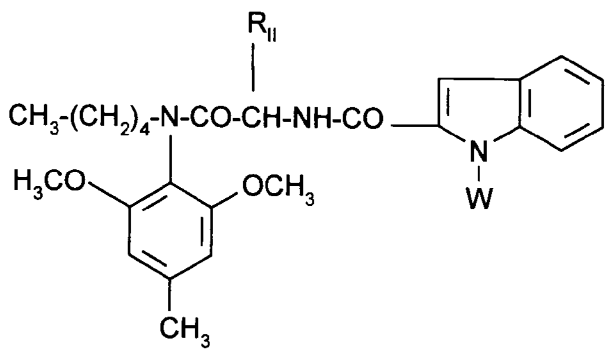

| Exemple Numéro | $R_{II}$ et configuration | W | F ; °C | $[\alpha]_D^{20}$ (c = ; solvant) |
|---|---|---|---|---|
| **8** | H | H | 180 | - |
| **9** | H | $-CH_2COOCH_3$ | 130 | - |
| **10** | H | $-CH_2COOH$ | 188 | - |
| **11** | H | $-CH_2CH_2COOH$ | 76 | - |
| **12** | $-CH_3$ (R) | $-CH_2COOCH_3$ | 73 | ND |

**TABLEAU I** : Suite 1

| Exemple Numéro | $R_{II}$ et configuration | W | F ; °C | $[\alpha]_D^{20}$ (c = ; solvant) |
|---|---|---|---|---|
| 13 | $-CH_3$ (R) | $-CH_2COOH$ | 98 | - 99,6 (1 ; $CH_3OH$) |
| 14 | $-CH_2OH$ (R) | H | 124 | - 48,4 (1 ; $CH_2Cl_2$) |
| 15 | $-CH_2COOH$ (R) | H | 205 | - 76,8 (1 ; $CHCl_3$) |
| 16 | $-CH_2CH_2COOH$ (R) | H | 110 | - 84,6 (0,9 ; DMF) |
| 17 | $-CH_2CH_2COOH$ (S) | H | 110 | + 67,6 (0,9 ; DMF) |
| 18 | 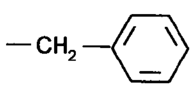 (R) | H | 252 | - 12,7 (1,05 ; $CH_2Cl_2$) |
| 19 | 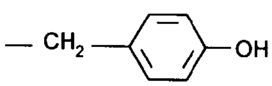 (R) | H | 204 | - 8,8 (0,99 ; DMF) |
| 20 | $-CH_2CH_2COOCH_2$ 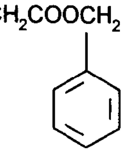 (S) | H | 124 | + 57,0 (1 ; DMF) |

**TABLEAU I** : Suite 2

| Exemple Numéro | $R_{II}$ et configuration | W | F ; °C | $[\alpha]_D^{20}$ (c = ; solvant) |
|---|---|---|---|---|
| 21 | 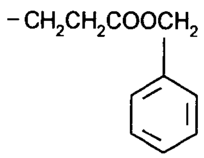 (R) | H | 128 | - 56,2 (1 ; DMF) |
| 22 | 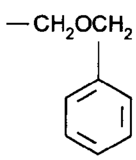 (R) | H | 179 | - 62,2 (1 ; $CH_2Cl_2$) |
| 23 | $-CH_2CONH_2$ (R) | H | 206 | insoluble |
| 24 | $-CH_2CH_2CONH_2$ (R) | H | 135 | - 99,8 (1,04 ; $CH_2Cl_2$) |
| 25 | 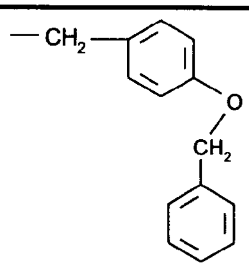 (R) | H | 193 | ND |
| 26 | $-CH_2OH$ (R) | $-CH_2COOH$ | 192 | - 36,6 (1 ; $CH_3OH$) |
| 27 | $-CH_2COOH$ (R) | $-CH_2COOH$ | 115 | - 85,0 (1 ; $CHCl_3$) |

**TABLEAU I** : Suite 3

| **Exemple Numéro** | **$R_{II}$ et configuration** | **W** | **F ; °C** | **$[\alpha]_D^{20}$ (c = ; solvant)** |
|---|---|---|---|---|
| **28** | $-CH_2CH_2COOH$ (R) | $-CH_2COOH$ | 100 | - 67,7 (0,9 ; DMF) |
| **29** | $-CH_2CH_2COOH$ (S) | $-CH_2COOH$ | 128 | + 73,3 (0,9 ; DMF) |
| **30** | 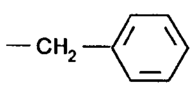 (R) | $-CH_2COOH$ | 115 | - 25,6 (1,02 ; $CH_3OH$) |
| **31** | 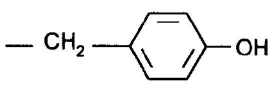 (R) | $-CH_2COOH$ | 238 | - 8,3 (1,02 ; DMF) |
| **32** | $-CH_2CONH_2$ (R) | $-CH_2COOH$ | 150 | - 62,7 (1 ; CH3OH) |
| **33** | $-CH_2CH_2CONH_2$ (R) | $--CH_2COOH$ | 150 | - 106,4 (1,17 ; $CH_2Cl_2$) |
| **34** | $-CH_2OH$ (R) | $-CH_2COOCH_3$ | 87 | - 62,2 (1 ; $CH_3OH$) |
| **35** | $-CH_2COOH$ (R) | $-CH_2COOCH_3$ | 104 | - 81,0 (0,99 ; $CHCl_3$) |

**TABLEAU I** : Suite 4

| Exemple Numéro | $R_{II}$ et configuration | W | F ; °C | $[\alpha]_D^{20}$ (c = ; solvant) |
|---|---|---|---|---|
| **36** | $-CH_2CH_2COOH$ (R) | $-CH_2COOCH_3$ | 80 | - 62,4 (0,9 ; DMF) |
| **37** | 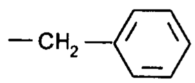 (R) | $-CH_2COOCH_3$ | 162 | - 21,8 ($CH_3OH$) |
| **38** | 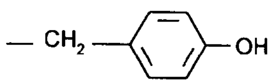 (R) | $-CH_2COOCH_3$ | 120 | - 10,6 (0,98 ; DMF) |
| **39** | 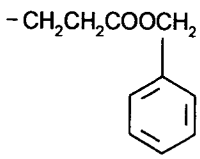 (R) | $-CH_2COOCH_3$ | 50 | - 47,3 (0,9 ; DMF) |
| **40** | 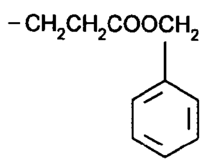 (S) | $-CH_2COOCH_3$ | 48 | + 47,0 (0,9 ; DMF) |
| **41** | $-CH_2CONH_2$ (R) | $-CH_2COOCH_3$ | 213 | - 115,2 (1 ; $CH_2Cl_2$) |
| **42** | $-CH_2CH_2CONH_2$ (R) | $-CH_2COOCH_3$ | 152 | - 80,8 (1,02 ; $CH_2Cl_2$) |

**TABLEAU I** : Suite 5

| **Exemple Numéro** | **$R_{II}$ et configuration** | **W** | **F ; °C** | **$[\alpha]_D^{20}$ (c = ; solvant)** |
|---|---|---|---|---|
| **43** | 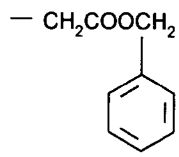 (R) | 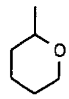 | 66 | -51,8 (1 ; $CHCl_3$) |
| **44** | 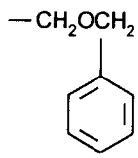 (R) | $-CH_2COONa$ | 154 | -35,8 (0,92 ; $CH_3OH$) |
| **45** | $-CH_3$ (S) | $-CH_2COOCH_3$ | 72 | +101,5 (1 ; $CH_3OH$) |
| **46** | $-CH_3$ (S) | $-CH_2COOH$ | 94 | +100,8 (1 ; $CH_3OH$) |
| **47** | $-CH_3$ (S) | H | 195 | +86,3 (1 ; $CH_2Cl_2$) |
| **48** | $-CH_3$ (R) | 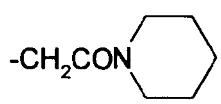 | 86 | -81,5 (0,998 ; $CH_3OH$) |

**TABLEAU I** : Suite 6

| Exemple Numéro | $R_{II}$ et configuration | W | F ; °C | $[\alpha]_D^{20}$ (c = ; solvant) |
|---|---|---|---|---|
| 49 | 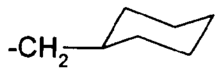 (R) | H | 242 | -29,7 (0,96 ; $CH_2Cl_2$) |
| 50 | 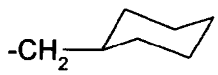 (R) | $-CH_2COOCH_3$ | 90 | -62,2 (1,1 ; $CH_2Cl_2$) |
| 51 | 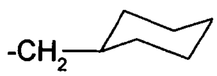 (R) | $-CH_2COONa$ | 220 | -40,2 (0,96 ; $CH_2Cl_2$) |
| 52 | 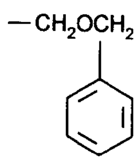 (S) | $-CH_2COOCH_3$ | 60 | +47,2 (0,94 ; $CH_3OH$) |
| 53 | 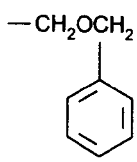 (S) | H | 176 | +47,8 (0,93 ; $CH_2Cl_2$) |
| 54 | $-CH_3$ (R) | $-CH_2CH_2COOH$ | 67 | -102,9 (0,998 ; $CH_3OH$) |
| 55 | $-CH_3$ (R) | $-CH_3$ | 67 | -114,0 (1 ; $CH_3OH$) |

**TABLEAU I** : Suite 7

| Exemple Numéro | $R_{II}$ et configuration | W | F ; °C | $[\alpha]_D^{20}$ (c = ; solvant) |
|---|---|---|---|---|
| **56** | 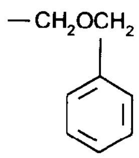 (S) | $-CH_2COOLi$ | 158 | +40,0 (1 ; $CH_3OH$) |
| **57** | $-CH_3$ (R) | $-CH_2CH_2OH$ | 66 | -108,9 (1 ; $CH_3OH$) |
| **58** | $-CH_3$ (R) | $-CH_2COOCH_3$ | 60 | -46,5 (1,1 ; $CH_3OH$) |
| **59** | 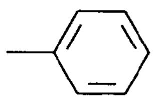 Racémique | $-CH_2COOCH_3$ | 75 | - |
| **60** | 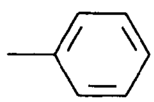 Racémique | H | 229 | - |
| **61** | 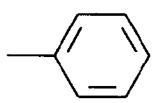 Racémique | $-CH_2COOH$ | 186 | - |

**TABLEAU I** : Suite 8

| Exemple Numéro | $R_{II}$ et configuration | W | F ; °C | $[\alpha]_D^{20}$ (c = ; solvant) |
|---|---|---|---|---|
| **62** | $-CH_3$ (R) | $-C_2H_5$ | 48 | -116,2 (1,005 ; $CH_3OH$) |
| **63** | $-CH(CH_3)_2$ (R) | H | 231 | -148,1 (1 ; DMF) |
| **64** | $-CH(CH_3)_2$ (R) | $-CH_2COOCH_3$ | 76 | -102,5 (1 ; $CH_3OH$) |
| **65** | $-CH(CH_3)_2$ (R) | $-CH_2COOH$ | 192 | -113,4 (1 ; $CH_3OH$) |
| **66** | $-(CH_2)_4NHCOOCH_2$ 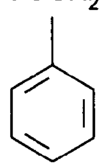 (R) | $-CH_2COOCH_3$ | 68 | -44,0 (1 ; $CH_3OH$) |
| **67** | $-(CH_2)_4NHCOOCH_2$ 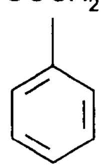 (R) | H | 95 | -63,3 (0,92 ; $CH_3OH$) |

**TABLEAU I** : Suite 9

| Exemple Numéro | $R_{II}$ et configuration | W | F ; °C | $[\alpha]_D^{20}$ (c = ; solvant) |
|---|---|---|---|---|
| **68** | $-(CH_2)_4NH_2$ (R) | H | 134 | -83,9 (0,9 ; $CH_3OH$) |
| **69** | $-(CH_2)_4NHCOOCH_2$ 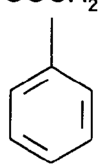 (R) | $-CH_2COOH$ | 118 | -46,5 (1 ; $CH_3OH$) |
| **70** | $-CH_2OCH_2$ 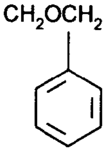 (R) | $-CH_2CH_2COOCH_3$ | huile | -46,7 (0,8 ; $CH_3OH$) |
| **71** | $-CH_2OCH_2$ 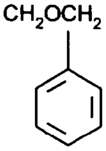 (R) | $-CH_2CH_2COOH$ | 84 | -45,0 (0,85 ; $CH_3OH$) |
| **72** | $-{}^*CH(CH_3)O-CH_2$ (S) (R) 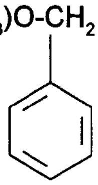 | $-CH_2COOCH_3$ | 117 | -95,0 (0,995 ; $CH_3OH$) |
| **73** | $-{}^*CH(CH_3)O-CH_2$ (S) (R) 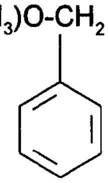 | $-CH_2COOH$ | 85 | -100,7 (1,01 ; $CH_3OH$) |

**TABLEAU I** : Suite 10

| Exemple Numéro | $R_{II}$ et configuration | W | F ; °C | $[\alpha]_D^{20}$ (c = ; solvant) |
|---|---|---|---|---|
| **74** | $-*CH(CH_3)O-CH_2$ (S) (R) 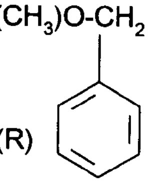 | H | 195 | -92,5 (1 ; $CH_2Cl_2$) |
| **75** | $-(CH_2)_4NH_2$ (R) | $-CH_2COOH$ | 173 | -173,0 (0,6 ; $CH_3OH$) |
| **76** | $—CH_2OCH_2$ 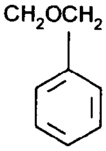 (R) | $-CH_2CH_3$ | huile | -48,3 (1 ; $CH_3OH$) |
| **77** | $—CH_2OCH_2$ 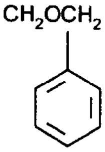 (R) | $-CH_3$ | 107 | -60,4 (1 ; $CH_3OH$) |
| **78** | $—CH_2SCH_2$ 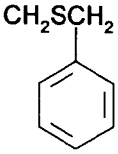 (R) | $-CH_2COOCH_3$ | 110 | -23,7 (1,007 ; $CH_3OH$) |
| **79** | $-CH_2CH_2$ 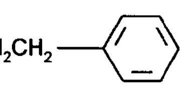 (R) | $-CH_2COOCH_3$ | 66 | -32,6 (0,995 ; $CH_3OH$) |

**TABLEAU I** : Suite 11

| Exemple Numéro | $R_{II}$ et configuration | W | F ; °C | $[\alpha]_D^{20}$ (c = ; solvant) |
|---|---|---|---|---|
| **80** | 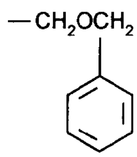 (R) | $-CH_2CH_2OH$ | 58 | -32,4 (1 ; $CH_3OH$) |
| **81** | 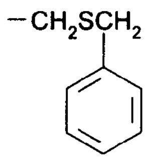 (R) | $-CH_2COOH$ | 72 | -20,6 (1 ; $CH_3OH$) |
| **82** | 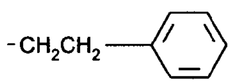 (R) | $-CH_2COOH$ | 101 | -34,0 (1 ; $CH_3OH$) |

## TABLEAU II

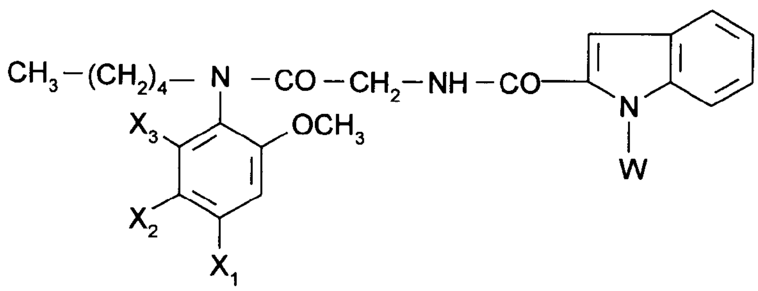

| Exemple numéro | $X_1$ | $X_2$ | $X_3$ | W | F ; °C |
|---|---|---|---|---|---|
| 83 | $OCH_3$ | $CH_3$ | Cl | $-CH_2COOCH_3$ | 129 |
| 84 | $OCH_3$ | $CH_3$ | Cl | H | 178 |
| 85 | $OCH_3$ | $CH_3$ | Cl | $CH_2COOH$ | 175 |
| 86 | Cl | $CH_3$ | H | $CH_2COOCH_3$ | 90 |
| 87 | Cl | $CH_3$ | H | H | 189 |
| 88 | Cl | $CH_3$ | H | $-CH_2COOH$ | 197 |
| 89 | Cl | $OCH_3$ | H | H | 194 |

**TABLEAU II** : Suite 1

| Exemple numéro | $X_1$ | $X_2$ | $X_3$ | W | F ; °C |
|---|---|---|---|---|---|
| 90 | Cl | $OCH_3$ | H | $-CH_2COOCH_3$ | 132 |
| 91 | Cl | $OCH_3$ | H | $-CH_2COOH$ | 118 |
| 92 | $CH_3$ | Cl | H | H | 196 |
| 93 | $CH_3$ | Cl | H | $-CH_2COOCH_3$ | 116 |
| 94 | $CH_3$ | Cl | H | $-CH_2-COO^-Na^+$ | 115 |

## TABLEAU III

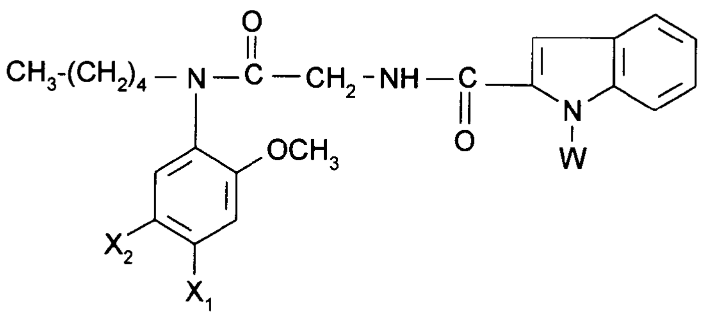

| Exemple numéro | $X_1$ | $X_2$ | W | F ; °C |
|---|---|---|---|---|
| 95 | $OCH_3$ | CH3 | H | 61 |
| 96 | $OCH_3$ | CH3 | $CH_2COOH$ | 130 |
| 97 | $CH_3$ | $OCH_3$ | H | 189 |
| 98 | $OCH_3$ | $OCH_3$ | $CH_2COOCH_3$ | 51 |
| 99 | $OCH_3$ | $OCH_3$ | $CH_2COOH$ | 202 |
| 100 | $OCH_3$ | $OCH_3$ | H | 199 |
| 101 | $CH_3$ | $OCH_3$ | $CH_2COOCH_3$ | 110 |

**TABLEAU III** : Suite 1

| Exemple numéro | $X_1$ | $X_2$ | W | F ; °C |
|---|---|---|---|---|
| 102 | $CH_3$ | $OCH_3$ | 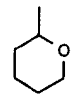 | 76 |
| 103 | $CH_3$ | $OCH_3$ | $CH_2COOH$ | 166 |

## TABLEAU IV

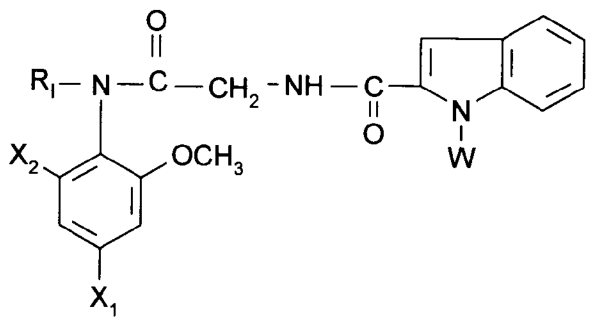

| Exemple numéro | $R_I$ | W | $X_1$ | $X_2$ | F ; °C |
|---|---|---|---|---|---|
| 104 | $-(CH_2)_6CH_3$ | H | $CH_3$ | $OCH_3$ | 129 |
| 105 | $-(CH_2)_6CH_3$ | $-CH_2COOH$ | $CH_3$ | $OCH_3$ | 178 |
| 106 | $-(CH_2)_6CH_3$ | $-(CH_2)_2COOCH_3$ | $CH_3$ | $OCH_3$ | 91 |
| 107 | $-(CH_2)_6CH_3$ | $-(CH_2)_2COOH$ | $CH_3$ | $OCH_3$ | 85 |
| 108 | $-(CH_2)_2CH(CH_3)_2$ | H | $CH_3$ | $OCH_3$ | 199 |
| 109 | $-(CH_2)_2CH(CH_3)_2$ | H | $OCH_3$ | $OCH_3$ | 188 |

**TABLEAU IV** : Suite 1

| Exemple numéro | $R_I$ | W | $X_1$ | $X_2$ | F ; °C |
|---|---|---|---|---|---|
| 110 | $-(CH_2)_2CH(CH_3)_2$ | $-CH_2COOH$ | $OCH_3$ | $OCH_3$ | 226 |
| 111 | 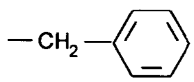 | H | $CH_3$ | $OCH_3$ | 225 |
| 112 | 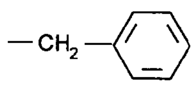 | $-CH_2COO^-Na^+$ | $CH_3$ | $OCH_3$ | 235 |
| 113 | 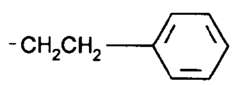 | H | $CH_3$ | $OCH_3$ | 238 |
| 114 | 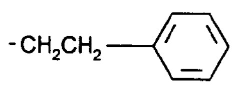 | $-CH_2COOCH_3$ | $OCH_3$ | H | 163 |
| 115 | 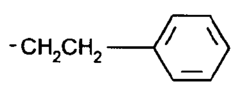 | $-CH_2COOH$ | $OCH_3$ | H | 198 |
| 116 | 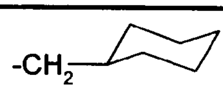 | H | $CH_3$ | $OCH_3$ | 234 |
| 117 | 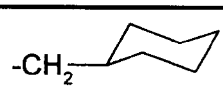 | $-CH_2COOCH_3$ | $CH_3$ | $OCH_3$ | 161 |

**TABLEAU IV** : Suite 2

| Exemple numéro | $R_I$ | W | $X_1$ | $X_2$ | F ; °C |
|---|---|---|---|---|---|
| 118 | $-CH_2$ 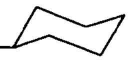 | $-CH_2COOH$ | $CH_3$ | $OCH_3$ | 212 |
| 119 | $-CH_2$ 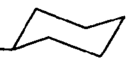 | $-CH_2CONH$<br>2 — Ada (1) | $CH_3$ | $OCH_3$ | 196 |
| 120 | $-(CH_2)_3OCH_3$ | H | $CH_3$ | $OCH_3$ | 198 |
| 121 | $-(CH_2)_3OCH_3$ | $-CH_2COOH$ | $CH_3$ | $OCH_3$ | 202 |
| 122 | $-(CH_2)_3OCH_3$ | $-CH_2COOCH_3$ | $CH_3$ | $OCH_3$ | 76 |
| 123 | $-(CH_2)_3OCH_3$ | $-(CH_2)_2COOH$ | $CH_3$ | $OCH_3$ | 103 |
| 124 | $-(CH_2)_3OCH_3$ | $-(CH_2)_2COOCH_3$ | $CH_3$ | $OCH_3$ | 94 |
| 125 | $-(CH_2)_2CH(CH_3)_2$ | $-CH_2COOH$ | $OCH_3$ | $OCH_3$ | 226 |

Note :
(1) 2-Ada représente le groupe 2-adamantyle

## TABLEAU V

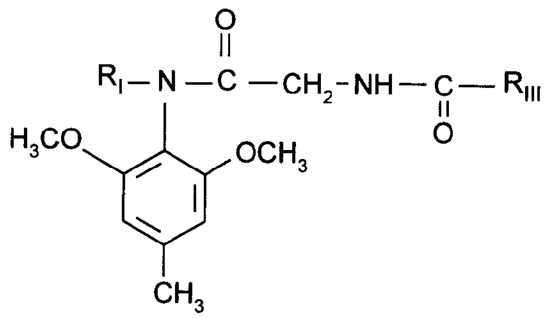

| Exemple numéro | $R_I$ | $R_{III}$ | F ; °C |
|---|---|---|---|
| 126 | $-(CH_2)_4CH_3$ | 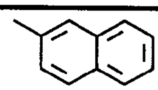 | 136 |
| 127 | $-(CH_2)_4CH_3$ | 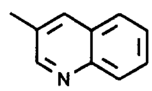 | 173 |
| 128 | $-(CH_2)_4CH_3$ | 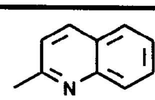 | 173 |
| 129 | $-(CH_2)_6CH_3$ | 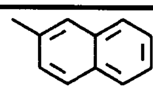 | 108 |
| 130 | $-(CH_2)_6CH_3$ | 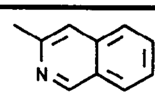 | 150 |
| 131 | $-(CH_2)_6CH_3$ | 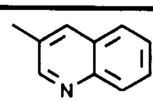 | 98 |
| 132 | $-(CH_2)_6CH_3$ | 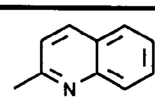 | 122 |

**TABLEAU V** : Suite 1

| Exemple numéro | $R_I$ | $R_{III}$ | F ; °C |
|---|---|---|---|
| **133** | $-(CH_2)_3OCH_3$ | 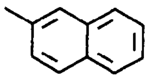 | 101 |
| **134** | $-(CH_2)_3OCH_3$ | 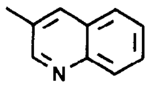 | 134 |
| **135** | $-(CH_2)_3OCH_3$ | 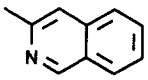 | 176 |
| **136** | $-(CH_2)_3OCH_3$ | 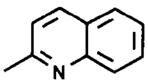 | 174 |
| **137** | $-(CH_2)_3OCH_3$ | 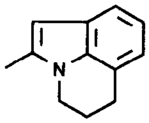 | 167 |

## TABLEAU VI

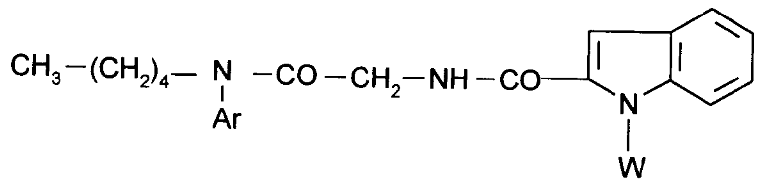

| Exemple numéro | Ar | W | F ; °C |
|---|---|---|---|
| 138 | 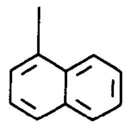 | $-CH_2COOCH_3$ | 71 |
| 139 | 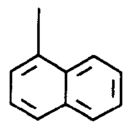 | $-CH_2COOH$ | 159 |

## TABLEAU VII

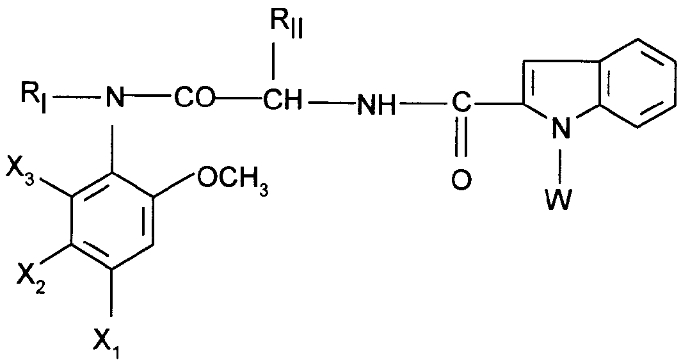

| Exemple numéro | $R_I$ | $R_{II}$ et configuration | $X_1$ | $X_2$ | $X_3$ | W | F ; °C $[\alpha]_D^{20}$ (c = ; solvant) |
|---|---|---|---|---|---|---|---|
| **140** | 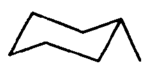 | $-CH_3$ (R) | $CH_3$ | H | $OCH_3$ | $-CH_2COOCH_3$ | 130<br>-87,4<br>(1 ; $CH_3OH$) |
| **141** | 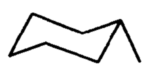 | $-CH_3$ (R) | $CH_3$ | H | $OCH_3$ | $-CH_2COOH$ | 171<br>-87,3<br>(1 ; $CH_3OH$) |
| **142** | 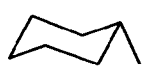 | $-CH_3$ (R) | $CH_3$ | H | $OCH_3$ | H | 252<br>-81,5<br>(1 ; $CH_2Cl_2$) |
| **143** | $-(CH_2)_4CH_3$ | $—CH_2OCH_2$<br>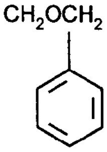 (R) | $OCH_3$ | $CH_3$ | Cl | H | 92<br>-74,4<br>(1,1 ; $CH_3OH$) |

**TABLEAU VII** : Suite 1

| Exemple numéro | $R_I$ | $R_{II}$ et configuration | $X_1$ | $X_2$ | $X_3$ | W | F ; °C $[\alpha]_D^{20}$ (c = ; solvant) |
|---|---|---|---|---|---|---|---|
| **144** | $-(CH_2)_4CH_3$ | 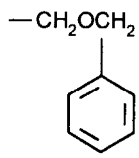 (R) | $OCH_3$ | $CH_3$ | H | $-CH_2COOCH_3$ | 78<br>-88,2<br>(1,4 ; $CH_3OH$) |
| **145** | $-(CH_2)_4CH_3$ | 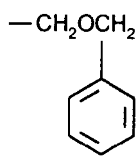 (R) | $OCH_3$ | $CH_3$ | Cl | $-CH_2COOH$ | 89<br>-59,3<br>(0,85 ; $CH_3OH$) |
| **146** | $-(CH_2)_4CH_3$ | 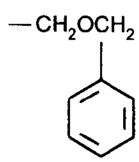 (R) | $OCH_3$ | $CH_3$ | H | H | 190<br>-104,0<br>(0,75 ; $CH_3OH$) |
| **147** | $-(CH_2)_4CH_3$ | 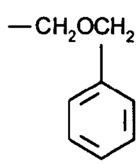 (R) | $OCH_3$ | $CH_3$ | H | $-CH_2COOH$ | 72<br>-66,5<br>(1 ; $CH_3OH$) |
| **148** | 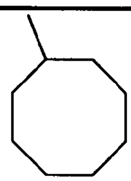 | 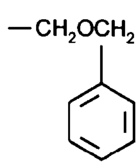 (R) | $CH_3$ | H | $OCH_3$ | $-CH_2COOH$ | 110<br>-54,0<br>(1,05 ; $CH_3OH$) |
| **149** | $-(CH_2)_4CH_3$ | 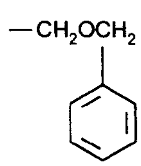 (R) | $OCH_3$ | $CH_3$ | Cl | $-CH_2COOCH_3$ | 55<br>-88,2<br>(1,1 ; $CH_3OH$) |

**TABLEAU VII** : Suite 2

| Exemple numéro | $R_I$ | $R_{II}$ et configuration | $X_1$ | $X_2$ | $X_3$ | W | F ; °C $[\alpha]_D^{20}$ (c = ; solvant) |
|---|---|---|---|---|---|---|---|
| 150 | 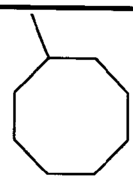 | 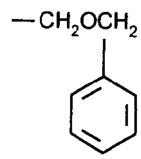 (R) | $CH_3$ | H | $OCH_3$ | $-CH_2CO_2CH_3$ | 78<br>-50,0<br>(0,985 ; $CH_3OH$) |
| 151 | $-(CH_2)_4CH_3$ | 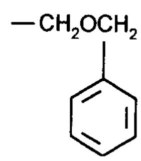 (R) | $CH_3$ | Cl | H | H | 186<br>-13,0<br>(1 ; DMF) |
| 152 | $-(CH_2)_4CH_3$ | 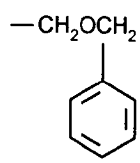 (R) | $CH_3$ | Cl | H | $-CH_2COOH$ | 205<br>-6,0<br>(1,1 ; DMF) |
| 153 | $-(CH_2)_4CH_3$ | 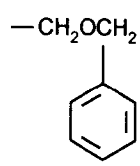 (R) | $CH_3$ | Cl | H | $-CH_2COOCH_3$ | 60<br>-6,0<br>(1,125 ; $CH_3OH$) |
| 154 | 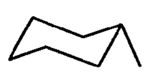 | 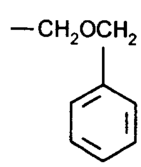 (R) | $CH_3$ | H | $OCH_3$ | $-CH_2COOCH_3$ | 80<br>-45,0<br>(1,07 ; $CH_3OH$) |
| 155 | 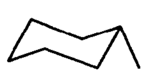 | 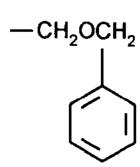 (R) | $CH_3$ | H | $OCH_3$ | $-CH_2COOH$ | 112<br>-45,0<br>(1 ; $CH_3OH$) |

**TABLEAU VII** : Suite 3

| Exemple numéro | $R_I$ | $R_{II}$ et configuration | $X_1$ | $X_2$ | $X_3$ | W | F ; °C $[\alpha]_D^{20}$ (c = ; solvant) |
|---|---|---|---|---|---|---|---|
| 156 | 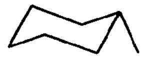 | $-CH_2OCH_2$ 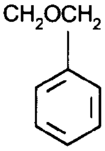 (R) | $CH_3$ | H | $OCH_3$ | H | 185 -70,0 (0,95 ; DMF) |
| 157 | $-CH_2CH_2-$ 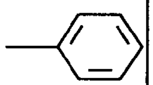 | $-CH_2OCH_2$ 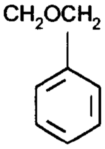 (R) | $CH_3$ | H | $OCH_3$ | $-CH_2COOCH_3$ | 68 -31,5 (1 ; $CH_3OH$) |
| 158 | $-CH_2CH_2-$ 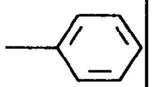 | $-CH_2OCH_2$ 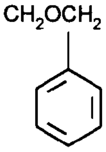 (R) | $CH_3$ | H | $OCH_3$ | H | 157 -33,0 (1 ; $CH_3OH$) |
| 159 | $-(CH_2)_4CH_3$ | $-CH_2OCH_2$ 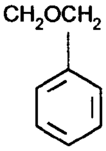 (R) | Cl | $CH_3$ | H | $-CH_2COOCH_3$ | 70 -19,0 (1,025 ; $CH_3OH$) |
| 160 | $-CH_2CH_2-$ 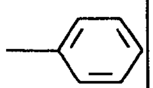 | $-CH_2OCH_2$ 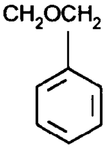 (R) | $CH_3$ | H | $OCH_3$ | $-CH_2COOH$ | 124 -33,0 (1 ; $CH_3OH$) |
| 161 | $-(CH_2)_4CH_3$ | $-CH_2OCH_2$ 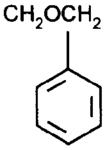 (R) | Cl | $CH_3$ | H | $-CH_2COOH$ | 135 -19,0 (1,15 ; $CH_3OH$) |

**TABLEAU VII** : Suite 4

| Exemple numéro | $R_I$ | $R_{II}$ et configuration | $X_1$ | $X_2$ | $X_3$ | W | F ; °C $[\alpha]_D^{20}$ (c = ; solvant) |
|---|---|---|---|---|---|---|---|
| 162 | $-(CH_2)_4CH_3$ | 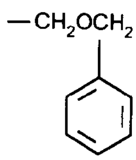 (R) | Cl | $CH_3$ | H | H | 186<br>-14,0<br>(0,95 ; $CH_2Cl_2$) |
| 163 | 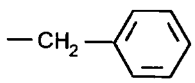 | 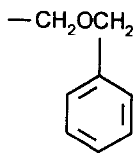 (R) | $CH_3$ | H | $OCH_3$ | $-CH_2COOCH_3$ | 73<br>-80,2<br>(1 ; $CH_3OH$) |
| 164 | $-(CH_2)_3CH_3$ | 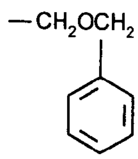 (R) | $CH_3$ | H | $OCH_3$ | $-CH_2COOCH_3$ | 60<br>-35,5<br>(1 ; $CH_3OH$) |
| 165 | 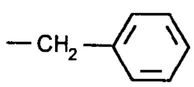 | 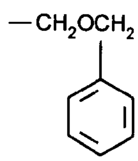 (R) | $CH_3$ | H | $OCH_3$ | $-CH_2COOH$ | 112<br>-79,5<br>(1 ; $CH_3OH$) |
| 166 | $-(CH_2)_4CH_3$ | 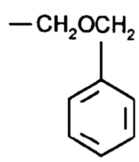 (R) | $OCH_3$ | H | $OCH_3$ | $-CH_2COOCH_3$ | 58<br>-58,3<br>(1 ; $CH_3OH$) |
| 167 | $-(CH_2)_4CH_3$ | 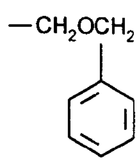 (R) | $OCH_3$ | H | $OCH_3$ | $-CH_2COOH$ | 99<br>-61,1<br>(1 ; $CH_3OH$) |

**TABLEAU VII** : Suite 5

| Exemple numéro | $R_I$ | $R_{II}$ et configuration | $X_1$ | $X_2$ | $X_3$ | W | F ; °C $[\alpha]_D^{20}$ (c = ; solvant) |
|---|---|---|---|---|---|---|---|
| 168 | 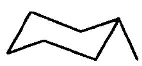 | 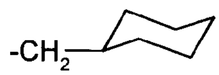 (R) | $CH_3$ | H | $OCH_3$ | $-CH_2COOH$ | 165 -52,0 (0,805; $CH_3OH$) |
| 169 | 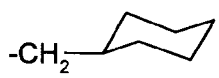 | 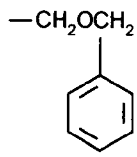 (R) | $CH_3$ | H | $OCH_3$ | $-CH_2COOCH_3$ | 130 -49,0 (0,9 ; $CH_3OH$) |
| 170 | 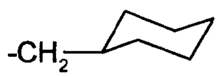 | 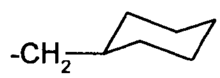 (R) | $CH_3$ | H | $OCH_3$ | $-CH_2COOCH_3$ | 120 -60,0 (0,97 ; $CH_3OH$) |
| 171 | 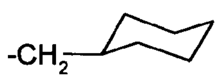 | 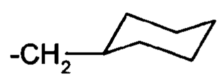 (R) | $CH_3$ | H | $OCH_3$ | $-CH_2COOH$ | 165 -55,0 (0,925 ; $CH_3OH$) |
| 172 | $-(CH_2)_3CH_3$ | 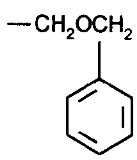 (R) | $CH_3$ | H | $OCH_3$ | $-CH_2COOH$ | 81 -55,1 (1 ; $CH_3OH$) |
| 173 | $-CH_2CH_3$ | 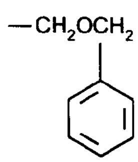 (R) | $CH_3$ | H | $OCH_3$ | $-CH_2COOCH_3$ | 71 -59,3 (1 ; $CH_3OH$) |

**TABLEAU VII** : Suite 6

| Exemple numéro | $R_I$ | $R_{II}$ et configuration | $X_1$ | $X_2$ | $X_3$ | W | F ; °C $[\alpha]_D^{20}$ (c = ; solvant) |
|---|---|---|---|---|---|---|---|
| 174 | $-CH_2CH_3$ | 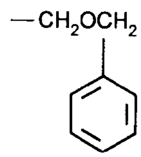 (R) | $CH_3$ | H | $OCH_3$ | $-CH_2COOLi$ | 100 -63,0 (1 ; $CH_3OH$) |
| 175 | 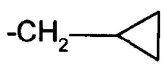 | 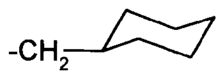 (R) | $CH_3$ | H | $OCH_3$ | $-CH_2COOCH_3$ | 107 -73,7 (1 ; $CH_3OH$) |
| 176 | 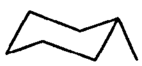 | 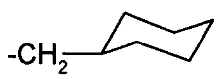 (R) | $CH_3$ | H | $OCH_3$ | $-CH_2COOCH_3$ | 150 -56,0 (0,925; $CH_3OH$) |
| 177 | $-(CH_2)_4CH_3$ | 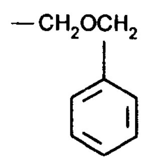 (R) | Cl | $OCH_3$ | H | $-CH_2COOCH_3$ | 70 -88,2 (0,97; $CH_3OH$) |
| 178 | 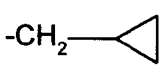 | 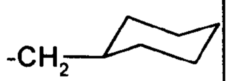 (R) | $CH_3$ | H | $OCH_3$ | $-CH_2COOH$ | 153 -78,4 (1; $CH_3OH$) |
| 179 | 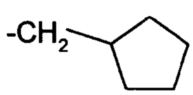 | 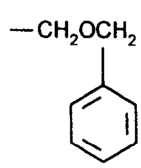 (R) | $CH_3$ | H | $OCH_3$ | $-CH_2COOCH_3$ | 74 -53,3 (1 ; $CH_3OH$) |

**TABLEAU VII** : Suite 7

| Exemple numéro | $R_I$ | $R_{II}$ et configuration | $X_1$ | $X_2$ | $X_3$ | W | F ; °C $[\alpha]_D^{20}$ (c = ; solvant) |
|---|---|---|---|---|---|---|---|
| 180 | $-CH_3$ | $-CH_2OCH_2$ 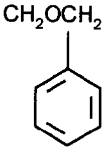 (R) | $CH_3$ | H | $OCH_3$ | $-CH_2COOCH_3$ | 64 -50,0 (1 ; $CH_3OH$) |
| 181 | $-CH_2$ 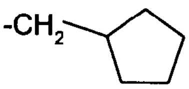 | $-CH_2OCH_2$ 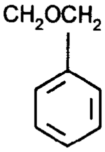 (R) | $CH_3$ | H | $OCH_3$ | $-CH_2COOH$ | 110 -36,8 (1 ; $CH_3OH$) |
| 182 | $-CH_2$ 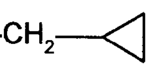 | $-CH_2OCH_2$ 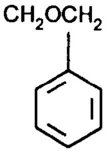 (R) | $CH_3$ | H | $OCH_3$ | $-CH_2COOH$ | 126 -65,0 (1 ; $CH_3OH$) |
| 183 | $CH_3$ | $-CH_2OCH_2$ 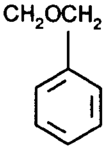 (R) | $CH_3$ | H | $OCH_3$ | $-CH_2COOH$ | 103 -58,6 (1 ; $CH_3OH$) |
| 184 | $-CH_2$ 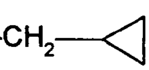 | $-CH_2OCH_2$ 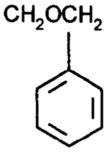 (R) | $CH_3$ | H | $OCH_3$ | $-CH_2COOCH_3$ | 76 -57,8 (1 ; $CH_3OH$) |

**EXEMPLE 185**

[2-{[butyl(2,6-diméthoxy-4-méthylphényl)carbamoyl]méthylcarbamoyl}-1-indolyl]acétate de méthyle.

(I) : Ar = 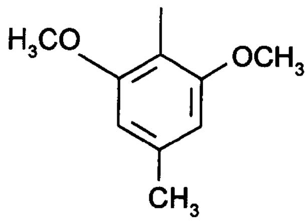 ; $R_I$ = $CH_3$— $(CH_2)_3$— ; $R_{II}$ = H ;

$R_{III}$ = 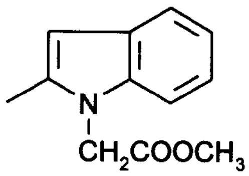

On prépare ce produit selon le procédé décrit à l'EXEMPLE 1 à partir de la butyl(2,6-diméthoxy-4-méthylphényl) carbamoylméthylamine et de l'acide 1-(méthoxycarbonylméthyl)-2-indolecarboxylique ; F = 139°C ; Rendement : 90 %.

**EXEMPLE 186**

(I) : Ar = 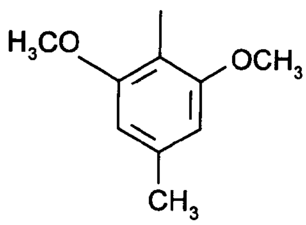 ; $R_I$ = $CH_3$— $(CH_2)_3$— ; $R_{II}$ = H ;

$R_{III}$ = 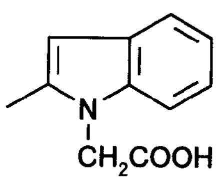

En procédant selon l'EXEMPLE 2, à partir du {2-[butyl(2,6-diméthoxy-4-méthylphényl)carbamoylméthylcarbamoyl]-1-indolyl}acétate de méthyle (EXEMPLE 185), on prépare l'acide {2- [[butyl(2,6-diméthoxy-4-méthylphényl)-carbamoyl]méthylcarbamoyl] 1-indolyl}acétique ; F = 211°C; Rendement : 92 %.

**EXEMPLE 187**

(I) : Ar = 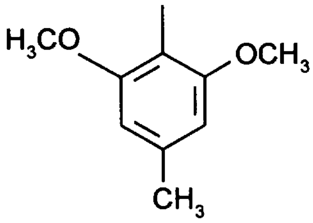 ; $R_I$ = 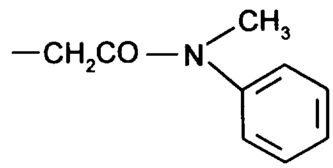 ; $R_{II}$ = H ;

$R_{III}$ = 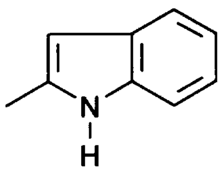

En procédant selon l'EXEMPLE 3, on prépare le N-{[((méthylphényl carbamoyl)méthyl)-(2,6-diméthoxy-4-méthylphényl)carbamoyl]méthyl}-1*H*-indole-2-carboxamide, F = 116°C ;
Rendement : 90 %.

**EXEMPLE 188**

(I) : Ar = 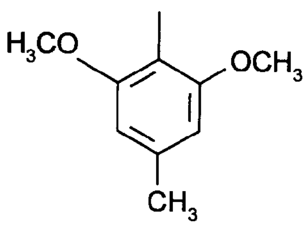 ; $R_I$ = $—(CH_2)_3CH_3$ ; $R_{II}$ = H ;

$R_{III}$ = 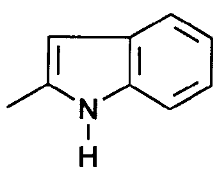

En procédant selon l'EXEMPLE 3, on prépare le N-[[butyl(2,6-diméthoxy-4-méthylphényl)carbamoyl]méthyl]-1*H*-indole-2-carboxamide ; F = 210°C ;
Rendement : 91 %.

**EXEMPLE 189**

(I) : Ar = 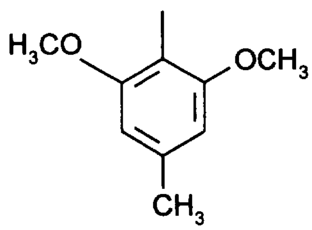 ; $R_I$ = — $(CH_2)_4CH_3$ ;

$R_{II}$ = — $(CH_2)_4NH_2$ $R_{III}$ = 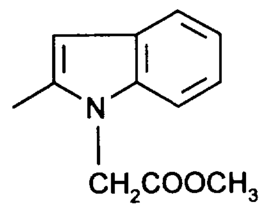

On dissout 5,6 g de (R)-{2-[N{1-[[(2,6-diméthoxy-4-méthylphényl)pentyl]-carbamoyl]-5-(benzyloxycarbonylamino)pentyl}-carbamoyl]-indol-1-yl}acétate de méthyle (EXEMPLE 66) dans 170 ml de méthanol et y ajoute 0,56 g de Pd/C à 10 %. On hydrogène sous une pression de 3 bars et abandonne le mélange réactionnel à 30°C en maintenant cette pression pendant 18 heures. Après refroidissement on filtre le catalyseur sur un lit de célite et évapore à sec. L'huile résiduelle est purifiée par chromatographie-flash sur gel de silice, éluant : $CH_2Cl_2/CH_3OH/AcOH$ 90/10/0,5 (v/v/v) pour obtenir des cristaux blancs de (R)-{2-[N-{1-[[(2,6-diméthoxy-4-méthylphényl)pentyl]-carbamoyl]-5-aminopentyl}-carbamoyl]-indol-1-yl}acétate de méthyle, F = 78°C,

$[\alpha]_D^{20}$ = -47,6° (c = 1, $CH_3OH$), Rendement : 85 %.

**EXEMPLE 190**

(I) : Ar = 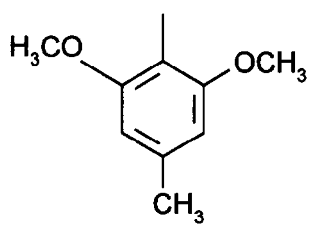 ; $R_I$ = — $(CH_2)_4CH_3$ ;

$R_{II}$ = — $(CH_2)_4NH_2$ $R_{III}$ = 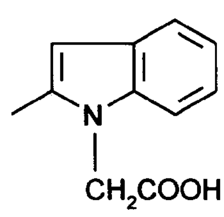

On dissout 0,7 g du composé préparé à l'EXEMPLE 189 précédent dans 20 ml de méthanol et ajoute 0,075 g d'hydroxyde de lithium hydraté et abandonne le mélange réactionnel à température ambiante pendant 18 heures. On évapore à sec et reprend le résidu par de l'eau. On acidifie la phase aqueuse par HCl 1N et extrait avec de l'acétate d'éthyle. Les extraits organiques sont séchés sur sulfate de sodium anhydre et évaporés à sec. Les cristaux obtenus sont purifiés par chromatographie-flash sur gel de silice, éluant : $CH_2Cl_2/CH_3OH$, 9/1 (v/v) pour obtenir des cristaux blancs de l'acide (R)-{2-[N-{1-[[(2,6-diméthoxy-4-méthylphényl)pentyl]-carbamoyl]-5-aminopentyl}-carbamoyl]-indol-1-yl)acétique, F = 173°C,

$[\alpha]_D^{20}$ = -173,0° (c = 0,6, $CH_3OH$), Rendement : 87 %.

**EXEMPLE 191**

(I) : Ar = 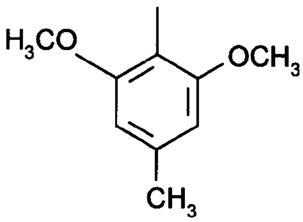 ; $R_{II}$ = —$(CH_2)_4NHCO$— 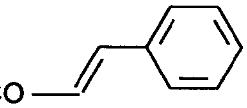

$R_{III}$ = 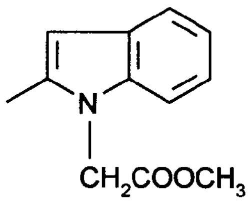 ; $R_I$ = —$(CH_2)_4CH_3$

A 30 ml de diméthylformamide, on ajoute successivement 0,87 g de (R)-{2-[N-{1-[[(2,6-diméthoxy-4-méthylphényl)pentyl]-carbamoyl]-5-aminopentyl}-carbamoyl]-indol-1-yl}acétate de méthyle, 0,645 g de BOP, 0,23 g d'acide cinnamique. Après refroidissement à -5°C, on ajoute sous atmosphère inerte 0,26 g de N-éthylmorpholine, on maintient le mélange réactionnel à 0°C pendant 2 heures puis abandonne à température ambiante pendant 18 heures. On verse le mélange réactionnel dans un grand volume d'eau et extrait avec de l'acétate d'éthyle. Les extraits organiques sont séchés sur sulfate de sodium anhydre et évaporés à sec. L'huile résiduelle est purifiée par chromatographie-flash sur gel de silice, éluant : $CH_2Cl_2/CH_3OH$ 97/3 (v/v) pour obtenir le (R)-{2-[N-{1-[[(2,6-diméthoxy-4-méthylphényl)pentyl]-carbamoyl]-5-(cinnamoylamino)pentyl}-carbamoyl]-indol-1-yl}acétate de méthyle sous forme d'huile.

EXEMPLE 192

(I) : Ar = 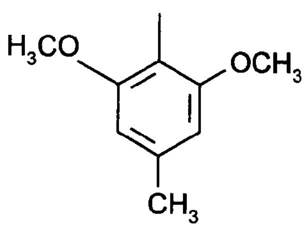 ; $R_{II}$ = —$(CH_2)_4NHCO$— 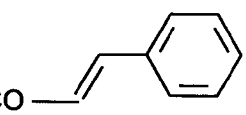

$R_{III}$ = 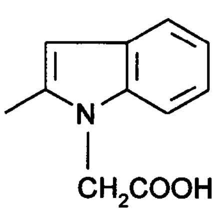 ; $R_I$ = —$(CH_2)_4CH_3$

L'ester précédent est saponifié avec $LiOH,H_2O/CH_3OH$ comme précédemment, pour fournir des cristaux blancs d' acide (R)-{2-[N-{1-[[(2,6-diméthoxy-4-méthylphényl)pentyl]-carbamoyl]-5-(cinnamoylamino)pentyl}-carbamoyl]-indol-1-yl} acétique, F = 172°C,

$[\alpha]_D^{20}$ = -21,2° (c = 0,8, $CH_3OH$).

**Revendications**

**1.** Composé de formule :

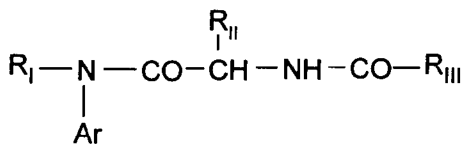

(I)

dans laquelle

- $R_I$ représente un alkyle en $C_3$ à $C_8$ ; un arylalkyle -Alk-$Ar_1$ où Alk représente un alkylène de 1 à 4 atomes de carbone et $Ar_1$ représente un groupe phényle ou un hétérocycle éventuellement substitué par un halogène, un alkyle en $C_1$-$C_3$, un alcoxy en $C_1$-$C_3$, un trifluorométhyle ou un hydroxyle ; un cycloalkylalkyle dans lequel l'alkyle est en $C_1$-$C_4$ et le cycloalkyle en $C_3$-$C_{10}$ ; un cycloalkyle en $C_3$-$C_{10}$ éventuellement substitué par un hydroxyle, un alcoxy en $C_1$-$C_3$ ou un alkyle en $C_1$-$C_3$ ledit alkyle pouvant substituer deux fois le même atome de carbone ; un alcoxyalkyle dans lequel l'alcoxy est en $C_1$-$C_4$ et l'alkyle est en $C_2$-$C_5$ ; ou un groupe (AB)N-CO-$(CH_2)_r$-, où A est un alkyle en $C_1$-$C_3$, B est un alkyle en $C_1$-$C_3$ ou un phényle ou bien A et B forment, avec l'atome d'azote auquel ils sont liés, un hétérocycle choisi parmi pyrrolidine, pipéridine et morpholine, r est 1, 2 ou 3 ;

- $R_{II}$ représente l'hydrogèn e; un alkyle en $C_1$-$C_6$ ; un hydroxyalkyle en $C_1$-$C_5$ ; un groupe $-(CH_2)_m-COR_2$ dans lequel m est un entier de 1 à 3 et $R_2$ représente un hydroxyle, un groupe alcoxy en $C_1$-$C_4$, un groupe benzyloxy, un groupe $-NR_3R_4$ dans lequel $R_3$ ou $R_4$ représentent indépendamment l'hydrogène, un alkyle en $C_1$-$C_4$ ou constituent avec l'atome d'azote auquel ils sont liés un hétérocycle choisi parmi pyrrolidine, pipéridine et morpholine ; un groupe aralkyle $-(CH_2)_n-Ar_2$ dans lequel n est égal à 0 ou représente un entier de 1 à 4 et $Ar_2$ représente un phényle ou un hétérocycle éventuellement substitué par un halogène, un alkyle en $C_1$-$C_3$, un alcoxy en $C_1$-$C_3$, un trifluorométhyle, un hydroxyle ou par un benzyloxy ; un cycloalkylalkyle dans lequel l'alkyle est en $C_1$-$C_4$ et le cycloalkyle en $C_3$-$C_{10}$ ; un aminoalkyle en $C_1$-$C_4$ ; un groupe R-CO-NH-$(CH_2)_x$- dans lequel x représente un nombre entier de 1 à 4 et R représente un alkyle en $C_1$-$C_4$, un phényle, un benzyle, un 2-phényléthényle ou un benzyloxy, les noyaux aromatiques étant éventuellement substitués par un halogène, un alkyle en $C_1$-$C_3$, un alcoxy en $C_1$-$C_3$, un trifluorométhyle, un hydroxyle, un groupe sulfonique ou carboxylique ; un guanidinoalkyle en $C_1$-$C_4$ ; un imidazolylalkyle en $C_1$-$C_3$ ; un alkylthioalkyle dans lequel les alkyles sont en $C_1$-$C_3$ ; un aralkylthioalkyle dans lequel la partie aryle est éventuellement hétérocyclique et les parties alkyle sont en $C_1$-$C_3$, l'aryle étant éventuellement substitué par un halogène, un alkyle en $C_1$-$C_3$, un alcoxy en $C_1$-$C_3$, un trifluorométhyle, un hydroxyle ; un benzyloxyalkyle dans lequel l'alkyle est en $C_1$-$C_3$ et le phényle éventuellement substitué par un halogène, un hydroxyle, un alcoxy en $C_1$-$C_3$, un alkyle en $C_1$-$C_3$, un trifluorométhyle, un nitrile, un nitro ;

- $R_{III}$ représente un groupe naphtyle ; un groupe quinoléinyle ; un groupe isoquinoléinyle ; un groupe indolyle non substitué, substitué sur un carbone, ou substitué sur l'azote par un groupe alkyle en $C_1$-$C_3$, alkylcarbonyle en $C_1$-$C_4$, par un groupe $-(CH_2)_p-COR_5$, p étant un entier de 0 à 4 et $R_5$ représentant $OR'_5$ ou $NR'_5R''_5$ avec $R'_5$ et $R''_5$ identiques ou non représentant l'hydrogène ou un alkyle en $C_1$-$C_4$ ou bien $R'_5$ et $R''_5$ forment ensemble avec l'atome d'azote auquel ils sont liés une pipéridine, par un hydroxyalkyle en $C_1$-$C_4$, par un alcoxyalkyle en $C_2$-$C_6$, par un cyanoalkyle en $C_2$-$C_4$, par un tétrahydropyranyle, par un adamantylaminocarbonylalkyle en $C_1$-$C_4$, ou par une chaîne $-(CH_2)_q-$, q étant un entier de 2 à 4 dont un des carbones substitue le noyau phényle du groupe indole pour constituer un cycle ;

- Ar représente un groupe 2-méthoxy-3-pyridinyle, 4-méthoxy-5-pyrimidinyle ou 2-méthoxyphényle contenant au moins deux autres substituants choisis parmi un alkyle en $C_1$-$C_3$, un alcoxy en $C_1$-$C_3$, un atome d'halogène et un trifluorométhyle ; ou Ar représente un groupe naphtyle ;

- ou bien $R_I$ et $R_{II}$ constituent ensemble un groupe

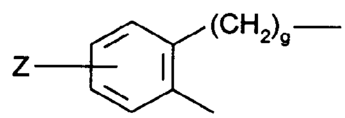

dans lequel g représente 0, 1 ou 2 et Z représente un alkyle en $C_1$-$C_4$, un alcoxy en $C_1$-$C_3$ ou un halogène ; ou éventuellement un de leurs sels.

**2.** Composé selon la revendication 1, de formule :

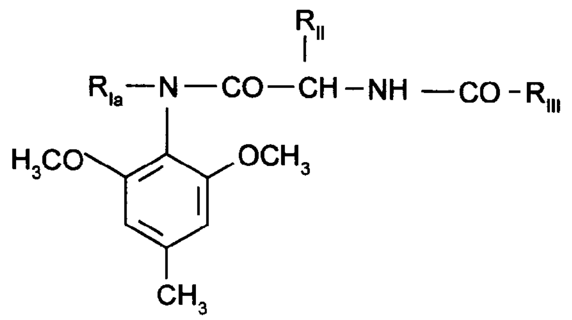

(Ia)

dans laquelle $R_{II}$ et $R_{III}$ sont tels que définis dans la revendication 1 pour (I) et $R_{Ia}$ représente un alkyle en $C_5$-$C_8$ ; un arylalkyle -Alk-$Ar_1$ où Alk représente un alkylène de 1 à 4 atomes de carbone et $Ar_1$ représente un groupe phényle ou un hétérocycle éventuellement substitué par un halogène, un alkyle en $C_1$-$C_3$, un alcoxy en $C_1$-$C_3$, un trifluorométhyle ou un hydroxyle ; un cycloalkylalkyle dans lequel l'alkyle est en $C_1$-$C_4$ et le cycloalkyle en $C_3$-$C_{10}$ ; un cycloalkyle en $C_3$-$C_{10}$ éventuellement substitué par un hydroxyle, un alcoxy en $C_1$-$C_3$ ou un alkyle en $C_1$-$C_3$ le dit alkyle pouvant substitué deux fois le même atome de carbone ; un alcoxyalkyle dans lequel l'alcoxy est en $C_1$-$C_4$ et l'alkyle est en $C_2$-$C_5$ ; ou un groupe (AB)N-CO-$(CH_2)_r$-, où A est un alkyle en $C_1$-$C_3$, B est un alkyle en $C_1$-$C_3$ ou un phényle ou bien A et B forment, avec l'atome d'azote auquel ils sont liés, un hétérocycle choisi parmi pyrrolidine, pipéridine et morpholine, r est 1, 2 ou 3 ; ou éventuellement un de leur sel.

**3.** Composé selon la revendication 1 de formule (I) dans laquel Ar représente un groupe naphtyle et $R_I$ est $R_{Ia}$ tel que défini dans la revendication 2 et ses sels éventuels.

**4.** Composé selon la revendication 1, de formule (I) dans laquelle $R_{II}$ est autre que de l'hydrogène et dans laquelle le carbone portant le substituant $R_{II}$ est en configuration R et ses sels éventuels.

**5.** Composé selon la revendication 1, de formule (I) dans laquelle $R_I$ et $R_{II}$ constituent ensemble un cycle

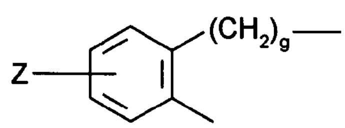

où g et Z sont tels que définis dans la revendication 1 et dans laquelle le carbone portant $R_{II}$ est en configuration S et ses sels éventuels.

**6.** Procédé pour la préparation d'un composé selon la revendication 1 de formule (I) caractérisé en ce que l'on traite une amine de formule :

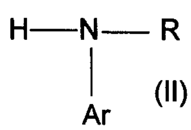

(II)

dans laquelle Ar et $R_I$ sont tels que définis ci-dessus, avec un aminoacide N-protégé de formule :

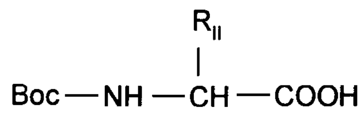

(III)

dans laquelle $R_{II}$ est tel que défini pour (I) et dans laquelle, le cas échéant, les fonctions réactives de $R_{II}$ ont été protégées pour conduire à un composé de formule :

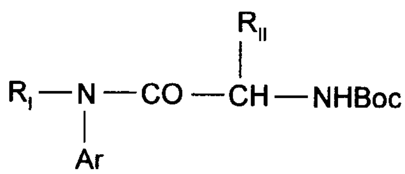

(IV)

dans laquelle $R_I$, Ar et $R_{II}$ sont tels que définis ci-dessus, lequel après transformation conduit à un composé (I) selon la revendication 1 ou l'un de ses sels éventuels.

**7.** Composé de formule :

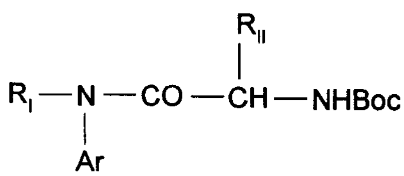

(IV)

dans lequel $R_I$, Ar et $R_{II}$ sont tels que définis dans la revendication 1.

**8.** Composition pharmaceutique contenant, en tant que principe actif, un composé selon l'une quelconque des revendications 1 à 5.

**9.** Composition pharmaceutique selon la revendication 8, sous forme d'unité de dosage, dans laquelle le principe actif est mélangé à au moins un excipient pharmaceutique.

**10.** Composition pharmaceutique selon la revendication 9, contenant de 0,5 à 1000 mg de principe actif.

**11.** Composé de formule :

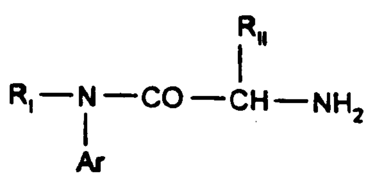

(V)

dans lequel $R_I$, Ar et $R_{II}$ sont tels que définis dans la revendication 1.

**12.** Composé de formule :

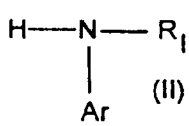

(II)

dans lequel Ar est tel que défini à la revendication 1 et $R_I$ représente un cycloalkyle en $C_3$-$C_{10}$ éventuellement substitué par un hydroxyle, un alcoxy en $C_1$-$C_3$ ou un alkykle en $C_1$-$C_3$ ledit alkyle pouvant substituer deux fois le même atome de carbone.

Office européen des brevets

# RAPPORT DE RECHERCHE EUROPEENNE

Numero de la demande
EP 95 40 1912

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | WO-A-91 13874 (RHONE-POULENC RORER S.A.)<br>* revendications *<br>--- | 1 | C07D209/42<br>A61K31/395<br>C07D405/04<br>C07D215/54<br>C07D215/48<br>C07D217/26<br>C07D487/06<br>C07C237/22 |
| D,A | JOURNAL OF MEDICINAL CHEMISTRY,<br>vol. 37, no. 5, 4 Mars 1994 WASHINGTON US,<br>pages 630-635,<br>M.W.HOLLADAY ET AL. 'Tetrapeptide CCK-A agonists: ...'<br>----- | | |

| DOMAINES TECHNIQUES RECHERCHES (Int.Cl.6) |
|---|
| C07D<br>A61K<br>C07C |

Le présent rapport a été établi pour toutes les revendications

| Lieu de la recherche | Date d'achèvement de la recherche | Examinateur |
|---|---|---|
| LA HAYE | 21 Novembre 1995 | Van Bijlen, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)